# EUROPEAN PATENT SPECIFICATION

(11) **EP 3 125 788 B1**
(45) Date of publication and mention of the grant of the patent: **02.09.2020**
(21) Application number: 15773794.1
(22) Date of filing: 04.04.2015
(51) Int. Cl.: A61B 10/00, A61B 17/16, A61B 10/02

(54) **COLLECTING AND HARVESTING CUT BONE FROM KERRISON RONGEUR**
SAMMLUNG UND GEWINNUNG VON GESCHNITTENEM KNOCHEN AUS KERRISON-RONGEUR
RECUEIL ET RÉCOLTE D'OS COUPÉ D'UN RONGEUR DE KERRISON

(30) Priority: 04.04.2014 US 201461975698 P
(43) Date of publication of application: 08.02.2017
(73) Proprietor: H & M Innovations, LLC, Wilmington, NC 28401-7908 (US)
(72) Inventor: STARKEY, Michael Morgan, Charlotte, NC 28210 (US); PHILPOTT, Thomas James, Charlotte, NC 28226 (US); HENSLER, Robert Sean, Wilmington, NC 28409 (US); MELIN, Thomas Eric, Wilmington, NC 28412 (US); MCNEIL, Raeshon Lamont, Charlotte, NC 28208 (US); GORMAN, Ryan Shane, Charlotte, NC 28226 (US)
(74) Representative: Swindell & Pearson Limited
(86) International application number: PCT/US2015/024402
(87) International publication number: WO 2015/154060

(56) References cited:
- WO-A1-2006/007455
- US-A- 5 422 273
- US-A1- 2001 005 786
- US-A1- 2011 202 063
- US-A1- 2013 345 596
- US-A1- 2014 074 240
- US-A1- 2014 209 119

## Description

### COPYRIGHT STATEMENT

All of the material in this patent document is subject to copyright protection under the copyright laws of the United States and other countries. The copyright owner has no objection to the facsimile reproduction by anyone of the patent document or the patent disclosure, as it appears in official governmental records but, otherwise, all other copyright rights whatsoever are reserved.

### BACKGROUND OF THE INVENTION

The present invention generally relates to the collection of cut material from a rongeur and, in particular, the collection of bone from a kerrison rongeur.

Rongeurs are surgical instruments for the cutting away of human tissue, and most commonly, cartilage and/or bone. "Kerrison" rongeurs are utilized in spinal surgery to remove bone and to thereby gain access to the spinal canal. rongeurs are well-known within conventional medical knowledge. Patent references disclosing and discussing kerrison rongeurs and their use in surgery include U.S. patents 3,902,498; 5,026,375; 4,722,338; 4,777,940; 4,777,948 and U.S. patent application publication 2003/0216740.

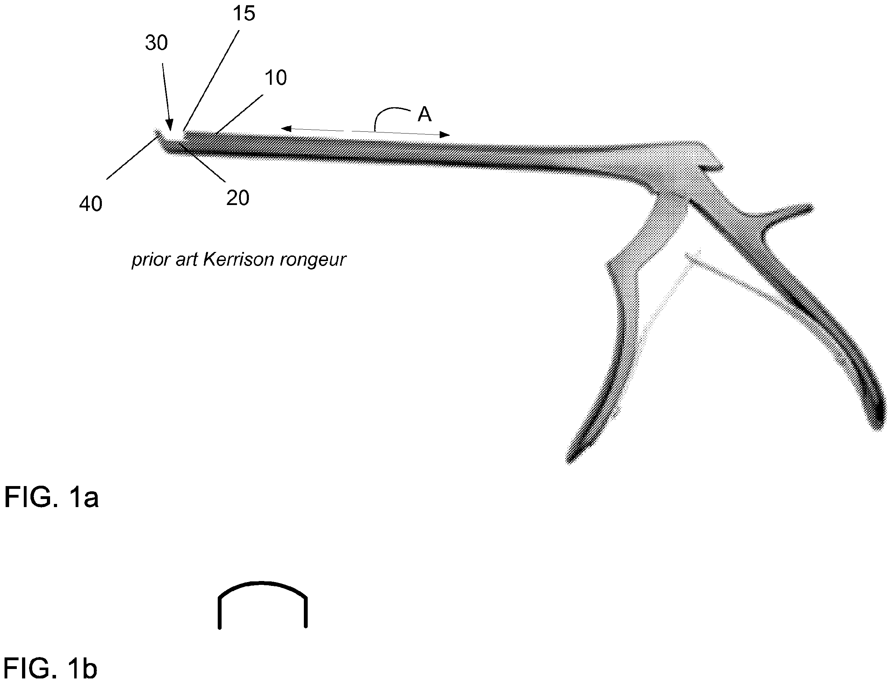

With reference to FIG. 1a, an exemplary prior art kerrison rongeur is illustrated. The kerrison rongeur includes a first jaw member **10** that slides parallel to line A relative to, and on top of, a second jaw member **20.** The first jaw member **10** included a distal cutting end having a cutting edge **15.** The second jaw member **20** includes a stop **40** for placement beneath the tissue to be cut, which is generally bone or cartilage. A cutting area **30** is defined between the cutting edge **15** of the first jaw member **10** and the stop **40** of the second jaw member **20.** The first jaw member **10** includes an open, interior cross-section defining an enclosed area within which the bone tissue is received when cut. Such an exemplary cross-section is illustrated in FIG. 1b. As illustrated, the cross-section of the first jaw member **10** has a generally inverted U shape.

In an exemplary use of a kerrison rongeur, a surgeon places the bone to be cut, such as the leading edge of the lamina of a vertebrae, within the open portion of the distal end of the rongeur. The surgeon then squeezes the handle of the rongeur, which advances a moveable jaw member of the rongeur through and amputates a portion of bone. A surgeon may wait until the jaw member becomes full of bone, at which time the rongeur must be completely removed from the surgical site and passed to a scrub nurse or assisting technician for bone removal. The removal from the instrument of the cut portion of bone often requires that the scrub nurse or assisting technician use a small rigid hook, or toothed forceps, and often further requires that the physician temporarily relinquish the instrument entirely to make such bone removal possible. Once cleaned, the instrument is returned to the surgeon who, in returning it to the surgical site, must then reorient himself to the task at hand. This sequence must then be repeated over and over again. Moreover, in a typical spinal fusion, this sequence may require as many as 50 to 100 such repetitions.

Alternatively, a surgeon may present the distal end of the rongeur to a scrub nurse or assisting technician after each cut for removal of the bone, thereby avoiding bone build-up within the jaw member of the rongeur. This can be done without the surgeon relinquishing the rongeur and without the surgeon removing his or her attention and focus from the surgical site. Conventionally, the scrub nurse or assisting technician manually swipes the end of the kerrison rongeur with a sterile material for removing the cut bone. The scrub nurse or assisting technician may repeat the swiping of the cut bone multiple times before harvesting the swiped bone from the material used to perform the swiping. A gauze sponge may be used for swiping. Rather than swiping the bone from the rongeur, a gloved hand may be used by the scrub nurse or assisting technician to directly remove the bone from the rongeur. Thereafter, the harvested bone from the patient may be used in the surgical procedure on the patient, e.g., in autografts.

In view of the foregoing, it is believed that a need exists for a safe, expedient, and efficient way for the cut bone to be removed from the kerrison rongeur and harvested by a scrub nurse or assisting technician without the surgeon relinquishing the kerrison rongeur and without the surgeon having to remove attention and focus from the surgical site. This, and other needs, are believed to be addressed by one or more aspects of the present invention.

WO2006007455 discloses a tissue collection lid for a specimen cup configured to prevent escape of fluid and/or tissue from the specimen cup during ejection of the fluid and/or tissue from a sampling instrument, such as a biopsy needle. The tissue collection lid includes a rim and a flange, the rim including an upper section and a lower section, the flange including a plurality of slits extending radially outward from an aperture in a mid-region thereof toward the rim. The rim defines an opening therethrough and the flange is positioned within the opening

### SUMMARY OF THE INVENTION

The present invention is as set out in the independent claims.

The present invention includes many aspects and features.

Accordingly, in one aspect of the present invention, a collector used to collect cut bone comprises: a container body defining an interior containment space for receiving and retaining collected bone, and having at least one open end for access and removal of collected bone from the interior containment space; and a cap in covering relation to the open end of the container body such that access to the interior containment space for removal of collected bone is inhibited. The collector comprises an intake port defining an opening for receiving therein a distal end of a kerrison-type rongeur for collecting cut bone from a cutting area thereof, and the cap comprises at least one scraper for engaging and dislodging cut bone from the cutting area of the distal end of the rongeur when received within the intake port.

In a feature of this aspect, the collector defines a passage through which cut bone dislodged by a scraper from the cutting area of the distal end of the rongeur is received within the interior containment space.

In additional features, the cap comprises a stop by which the extent to which the distal end of the rongeur received within the intake port is limited; and the stop is located at a spacing relative to the intake port such that, upon receipt of the distal end of the rongeur and abutment thereof with the stop, the one or more scrapers when actuated extend over and cover the distal end of the rongeur proximate the cutting area in which cut bone would be found.

In another feature, the container body comprises a stop by which the extent to which the distal end of the rongeur received within the intake port is limited.

In additional features, one or more of which may or may not be mutually exclusive: the collector further comprises a stop by which the extent to which the distal end of the rongeur received within the intake port is limited; the stop comprises a depressible button; the depressible button, when depressed, blocks the passage and inhibits receipt of dislodged bone within the interior containment space; and wherein the depressible button, when not depressed, does not inhibit receipt of dislodged bone within the interior containment space; the stop comprises a wall; the wall blocks the passage and inhibits receipt of dislodged bone within the interior containment space; and the wall includes an opening therein, the wall being movable between a first position in which the opening aligns with the passage and the wall does not inhibit receipt of dislodged bone within the interior containment space, dislodged bone passing through the opening in the wall, and a second position in which the opening is out of alignment with the passage and the wall inhibits receipt of dislodged bone within the interior containment space.

In additional features, one or more of which may or may not be mutually exclusive: the cap is detachable from the container body such that access to the interior space for removal of collected bone retained therein is uninhibited by the cap; the cap is attached to the container body in a frictional fit engagement; the cap is attached to the container body in a thread engagement; and the cap screws onto the container body.

In another feature, a portion of the cap is rotatable relative both to the container body and to another portion of the cap.

In additional features, one or more of which may or may not be mutually exclusive: the cap is movable relative to the container body detachable from the container body such that access to the interior space for removal of collected bone retained therein is uninhibited by the cap; a hinge connects the cap to the container body for movement relative thereto; the collector is disposable; one or more components of the collector are disposable; and one or more components of the collector are designed to be sterilized for reuse with different patients in different procedures.

In another feature, the collector is configured to collect cut bone from a kerrison rongeur.

In another feature, the collector is hand-held and lightweight.

In additional features, one or more of which may or may not be mutually exclusive: the container body comprises a generally elongate body or tube having opposite open-ends, wherein the cap comprises a first end cap covering a first of the opposite open-ends of the container body, and further comprising a second end cap covering a second of the opposite open-ends of the container body; and the first and second end caps are generally cylindrical in shape and are axially-aligned along a longitudinal axis of the collector.

In another feature, the one or more scrapers comprise a single scraper, the only one of which that is included in the collector.

In another feature, the one or more scrapers comprise more than one scraper.

In another feature, one of the one or more scrapers comprises bristles.

In another feature, one of the one or more scrapers comprises a brush.

In another feature, one of the one or more scrapers comprises a protuberance.

In another feature, one of the one or more scrapers comprises a barb.

In another feature, one of the one or more scrapers comprises a finger.

In another feature, one of the one or more scrapers is flexible and resilient, and sufficiently rigid so as to hold form when not engaged by the distal end of the rongeur and deflecting and bending upon abutment by and engagement with the distal end of the rongeur.

In another feature, one of the one or more scrapers is made from metal.

In another feature, one of the one or more scrapers is made from polypropylene.

In another feature, the one or more scrapers extend from an underside of the cap toward the interior containment space.

In another feature, the one or more scrapers not are located within the intake port.

In additional features, one or more of which may or may not be mutually exclusive: the one or more scrapers are located within the intake port; and the intake port is squeezable and springy such that the intake port can be squeezed by hand causing the one or more scrapers to enter to cutting area of a kerrison rongeur received within the intake port for dislodging bone carried therein.

In another feature, the container body comprises an opaque wall portion and a transparent wall portion.

In additional features, one or more of which may or may not be mutually exclusive: the container body comprises a movable panel; the panel is connected by a hinge for pivoting movement; and the panel is removable.

In another feature, the intake port is located in the container body.

In another feature, the intake port is located in a side wall of the container body generally halfway in-between opposite ends of the container body.

In another feature, the intake port is located in the cap.

In another feature, the intake port comprises a large, wide opening in the side of the cap configured to receive the tip of a rongeur of various conventional sizes.

In additional features, one or more of which may or may not be mutually exclusive: the intake port is located on the top of the cap; the top of the cap defines a funnel-shaped surface with the intake opening at the center thereof; the top of the cap defines a flange for catching dislodged bone when the collector is turned sideways and the distal end of the rongeur is received within the intake port; the cap defines a cutout for visual alignment of the collector when receiving the distal end of the rongeur; and the cap comprises a protuberance for visual alignment of the collector when receiving the distal end of the rongeur.

In another feature, the intake port is located on a side of the cap.

In another feature, the intake port includes a hawk bill profile.

In additional features, the intake port defines a scraping tip; and the scraping tip is rigid.

In another feature, the intake port includes an angular profile.

In another feature, the intake port includes a rounded profile.

In additional features, one or more of which may or may not be mutually exclusive: the one or more scrapers are manually movable into a position for engaging and dislodging cut bone from the cutting area of the distal end of the rongeur when received within the intake port; the one or more scrapers comprise at least two opposed scrapers that are manually moved in directions toward one another so as to converge within the cutting area of the distal end of the rongeur when received within the intake port; opposite ends of the collector each includes a depressible portion by which, when depressed, the opposed scrapers are manually moved so as to converge within the cutting area of the distal end of the rongeur when received within the intake port; the depressible portion is spring-biased against depression; the opposed scrapers are rotatable about a longitudinal axis of the container body by manual rotation of the depressible portions of the end caps; and the one or more scrapers comprise a scraper that is manually moved within the cutting area of the distal end of the rongeur when received within the intake port by twisting of the cap on the container body.

In additional features, one or more of which may or may not be mutually exclusive: the cap further comprises a handle; and the handle extends generally downwardly proximate a side of the container body, a distal end of the handle being located closer to an end of the container body opposite the cap.

In additional features, one or more of which may or may not be mutually exclusive: the collector further comprises a suction port for suctioning dislodged bone into receipt within the interior containment space of the container body; the cap defines the suction port; the suction port is located on a side of the cap; the cap further comprises a handle; the suction port is located on the handle; the handle extends generally downwardly proximate a side of the container body, a distal end of the handle being located closer to an end of the container body opposite the cap; and the suction port is located at the distal end of the handle, with an interior passage extending through the handle from the suction port and opens into the interior containment space defined by container body.

In another feature, the collector is configured to be held in a sideways position when used to collect cut bone from a kerrison rongeur.

In yet another feature, the container body includes an interior containment space having a graduated containment volume of thirty cubic centimeters.

In another feature, the collector is configured to be held in a horizontal position when used to collect cut bone from a kerrison rongeur, the cap and the container body being horizontally oriented relative to one another.

In another feature, the collector is configured to be held in an upright position when used to collect cut bone from a kerrison rongeur.

In another feature, the collector is configured to be held in a vertical position when used to collect cut bone from a kerrison rongeur, the cap and the container body being vertically oriented relative to one another with the cap being at a vertical elevation greater than the container body.

In another feature, the container body is box-shaped with generally rectangular sides and wherein the cap comprises rectangular sides.

In another feature, the container body is transparent. Preferably the container body is made from an inert material conventionally used with medical containers for holding human tissue for use in a body. In at least some embodiments, the container body comprises a molded plastic body, and the cap comprises a molded plastic body.

In another aspect, a device for collecting autologous bone fragments comprises a container and a cap. The container comprise a visibly transparent material and may include graduations to indicate volume of collected tissue comprising bone in the container. The cap is attachable to the container by way of threads on both the container and the cap, whereby the cap screws onto the container. Preferably the cap is symmetrical with the exception of an opening that is wide with respect to the diameter of the cap. The opening is configured to allow the passage of the tip of a kerrison rongeur of multiple sizes along with bone and other tissue matter carried on the tip. The cap further comprises a brush disposed on the underside of the cap facing the interior of the container. The brush comprises a plurality of bristles which may comprise individual monofilament bodies. Moreover, the bristles preferably comprise a material that is absorbable by the human body; a bio-absorbable bristle is preferred in the event that a bristle becomes dislodged and mixed with the harvested matter from the tip of the kerrison rongeur, and thereafter is inadvertently inserted into the patient. In particular, in use the tip of the kerrison rongeur carrying the matter cut from the patient is inserted through the opening of the cap, and the brush is used to dislodge the matter from the kerrison tip whereby the matter falls into the interior space of the container and is thereby collected. Dislodging the matter may be effected by moving the kerrison tip upward away from the container and into the brush in the underside of the cap and/or translating the kerrison tip perpendicularly to the length of the kerrison body. Additional or alternative movements can be used such as , for example, rotating the brush relative to the tip of the kerrison rongeur for dislodging the cut matter from the tip. The kerrison rongeur thereafter is removed for further use. Later when the harvested matter is needed, the cap is unscrewed from the container and the matter, i.e., bone in preferred implementations, is retrieved from the container.

In another aspect, a kit comprises an aforementioned collector and a rongeur, wherein the collector is configured to collect cut bone from the rongeur of the kit.

Another aspect comprises a method of using an aforementioned collector to collect cut bone from a kerrison rongeur.

In addition to the aforementioned aspects and features of the present invention, it should be noted that the present invention further encompasses the various possible combinations und of such aspects and features. Thus, for example, any aspect may be combined with an aforementioned feature in accordance with the present invention without requiring any other aspect or feature.

### BRIEF DESCRIPTION OF THE DRAWINGS

One or more preferred embodiments of the present invention now will be described in detail with reference to the accompanying drawings.
FIG. 1a is a side elevational view of an exemplary prior art kerrison rongeur.
FIG. 1b illustrates a cross-sectional shape of the first jaw member 10 of the kerrison rongeur of FIG. 1a.
FIG. 2a is a perspective view of a collector used to collect cut bone from a kerrison rongeur in accordance with a first embodiment of the present invention, wherein the collector is illustrated in a generally vertical orientation.
FIG. 2b is a perspective view illustrating a sequence of steps for using the collector of FIG. 2a in collecting bone from a kerrison rongeur, wherein the collector is illustrated in a generally horizontal orientation.
FIG. 2c is a perspective view of a portion of the collector of FIG. 2a after bone has been collected from a kerrison rongeur.
FIG. 3a is a perspective view of a collector used to collect cut bone from a kerrison rongeur in accordance with a second embodiment of the present invention, wherein the collector is illustrated in a generally vertical orientation.
FIG. 3b is a perspective view illustrating a sequence of steps for using the collector of FIG. 3a in collecting bone from a kerrison rongeur, wherein the collector is illustrated in a generally horizontal orientation.
FIG. 3c is a top plan view of a cap of the collector of FIG. 3a.
FIG. 3d is a side elevational view of the cap of the collector of FIG. 3a.
FIG. 4a is a perspective view representative of a sequence of steps for using another collector in accordance with a third embodiment of the present invention, wherein the collector is illustrated in a generally horizontal orientation receiving the distal end of a kerrison rongeur.
FIG. 4b is a top plan view of the collector and distal tip of the kerrison rongeur of FIG. 4a.
FIG. 5a a perspective view of a collector used to collect cut bone from a kerrison rongeur in accordance with another embodiment of the present invention, wherein the collector is illustrated in a generally vertical orientation.
FIG. 5b is a perspective view illustrating a sequence of steps for using the collector of FIG. 5a in collecting bone from a kerrison rongeur, wherein the collector is illustrated in a generally horizontal orientation with the distal end of the kerrison rongeur being received within the collector.
FIG. 5c is a side elevational view of an alternative embodiment to that of FIG. 5a, in which the height of the collector is less than that shown in FIG. 5a, the collector of FIG. 5c thus having a shorter profile.
FIG. 6a is a perspective view of a collector used to collect cut bone from a kerrison rongeur in accordance with another embodiment of the present invention, wherein the collector is illustrated in a generally horizontal orientation.
FIG. 6b is a side elevational view of the collector of FIG. 6a.
FIG. 7a is a perspective view of a collector used to collect cut bone from a kerrison rongeur in accordance with another embodiment of the present invention, wherein the collector is illustrated in a generally horizontal orientation.
FIG. 7b is a side elevational view of the collector of FIG. 7a.
FIG. 7c is another side elevational view of the collector of FIG. 7a.
FIG. 8a is a perspective view of a collector used to collect cut bone from a kerrison rongeur in accordance with another embodiment of the present invention, wherein the collector is illustrated in a generally horizontal orientation.
FIG. 9a is a perspective view of a collector used to collect cut bone from a kerrison rongeur in accordance with another embodiment of the present invention, wherein the collector is illustrated in a generally horizontal orientation.
FIG. 9b is a perspective view of part of a release mechanism of the collector of FIG. 9a.
FIG. 9c is a schematic illustration representing the arrangement and operation of the release mechanism of FIG. 9b.
FIG. 10a is a perspective view of a collector used to collect cut bone from a kerrison rongeur in accordance with yet another embodiment of the present invention, wherein the collector is illustrated in a generally vertical orientation and includes a suction tube attached thereto.
FIG. 10b is a side plan view of the collector of FIG. 10a and includes a close-up, cross-sectional view of an intake port of the collector of FIG. 10a within which a distal tip of a kerrison rongeur is received.
FIG. 11a is a perspective view of a collector used to collect cut bone from a kerrison rongeur in accordance with yet another embodiment of the present invention, wherein the collector is illustrated in a generally vertical orientation and includes a suction tube attached thereto.
FIG. 11b is a side plan view of the collector of FIG. 11a.
FIG. 12a is a side plan view in cross-section of a collector used to collect cut bone from a kerrison rongeur in accordance with yet another embodiment of the present invention, wherein the collector is illustrated in a generally vertical orientation and includes a suction tube attached thereto, and wherein the collector includes multiple interior scrapers.
FIG. 12b is a side plan view in cross-section of another collector similar to that of FIG. 12a, and differs in that the collector of FIG. 12b includes a single interior scraper.
FIG. 12c is a perspective view in cross-section of a portion of the collector of FIG. 12a illustrating a screen for filtering of outflow from the collector through the suction tube.
FIG. 13a is a perspective view of a collector used to collect cut bone from a kerrison rongeur in accordance with yet another embodiment of the present invention, wherein the collector is illustrated in a generally vertical orientation.
FIG. 13b is another perspective view of an opposite side of the collector as seen in FIG. 13a.
FIG. 14a is a perspective view of a collector used to collect cut bone from a kerrison rongeur in accordance with yet another embodiment of the present invention, wherein the collector is illustrated in a generally vertical orientation.
FIG. 14b is a perspective view illustrating a use of the collector of FIG. 14a in collecting bone from a kerrison rongeur, wherein the collector is illustrated in a generally horizontal orientation with the distal end of the kerrison rongeur about to be received through an intake port of the collector.
FIG. 15a is a perspective view of a collector used to collect cut bone from a kerrison rongeur in accordance with yet another embodiment of the present invention, wherein the collector is illustrated in a generally vertical orientation.
FIG. 15b is another perspective view of an opposite side of the collector as seen in FIG. 15a, wherein the intake port of the collector is better seen.
FIG. 16a is a perspective view of a collector used to collect cut bone from a kerrison rongeur in accordance with yet another embodiment of the present invention, wherein the collector is illustrated in a generally vertical orientation.
FIG. 16b is another perspective view of an opposite side of the collector as seen in FIG. 16a, wherein the intake port of the collector is better seen.
Each of FIGS. 17a-17f illustrates in elevational plan view, taken in cross-section, a shape of an intake port for a collector in accordance with various embodiments of the present invention.
Each of FIGS. 18a-18c illustrates in plan view an opening of an intake port for a collector in accordance with various embodiments of the present invention.
Each of FIGS. 19a-19c illustrates in plan view a generally oval (preferably circular) intake port opening of an intake port for a collector in accordance with various embodiments of the present invention.

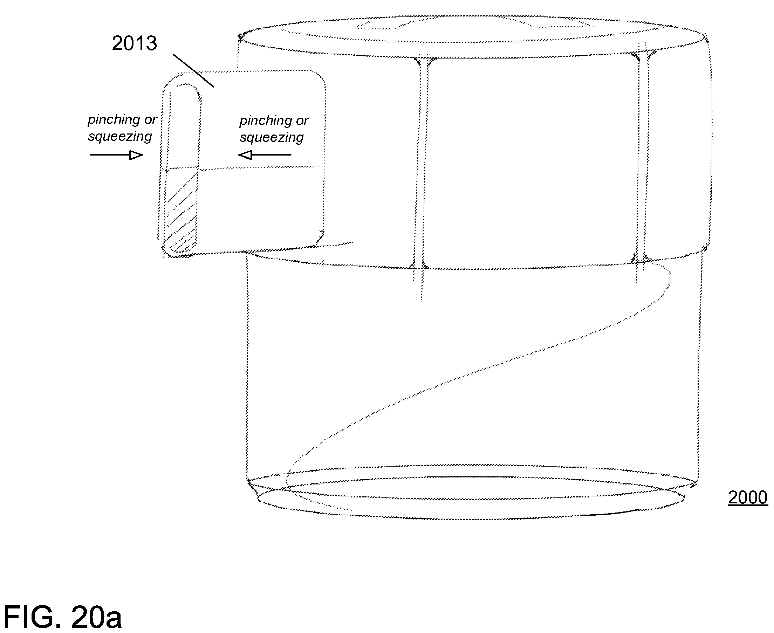

FIG. 20a is a perspective view of a collector used to collect cut bone from a kerrison rongeur in accordance with yet another embodiment of the present invention, wherein the collector is illustrated in a generally vertical orientation.

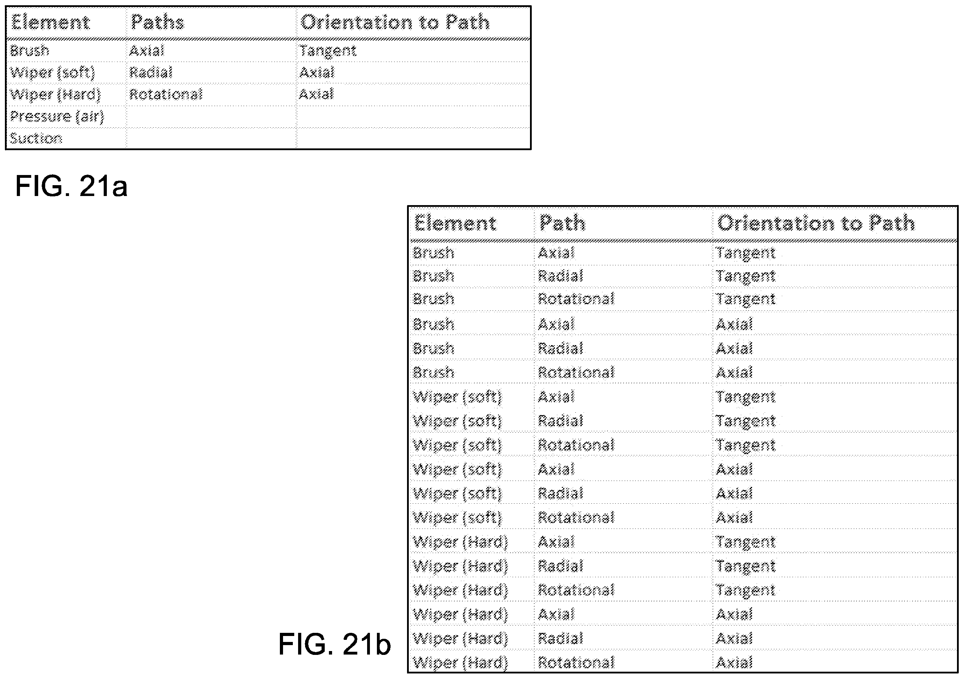

FIG. 21a illustrates a table listing values of properties giving rise to some possible variations between some embodiments of the invention.
FIG. 21b illustrates a table listing some possible combinations of some of the elements of the table of FIG. 21a.

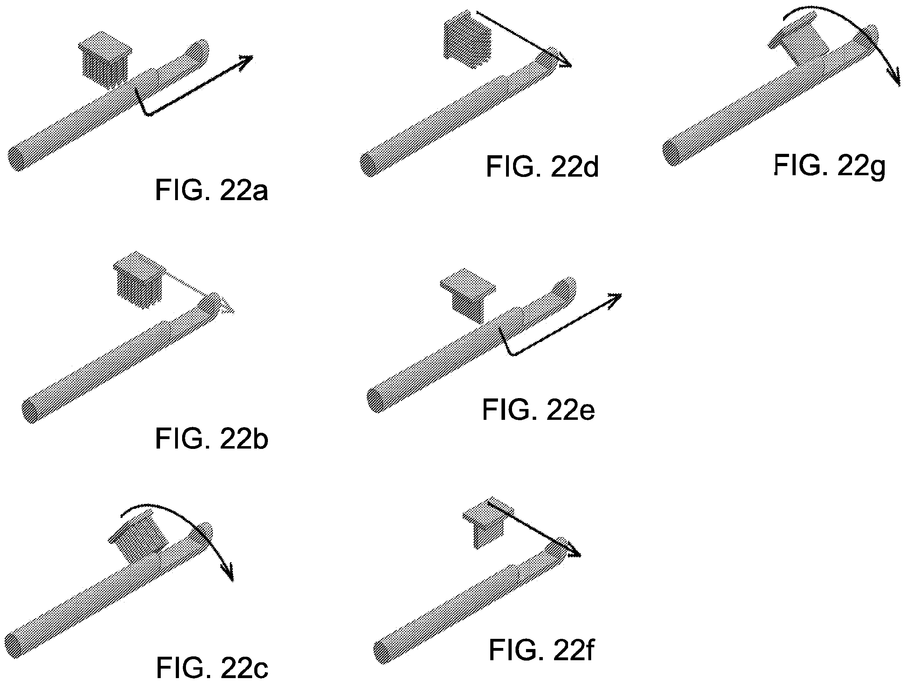

FIGS. 22a is a perspective view of a tip and scraper showing path and orientation properties representative of some embodiments of the invention.
FIGS. 22b is a perspective view of a tip and scraper showing path and orientation properties representative of some embodiments of the invention.
FIGS. 22c is a perspective view of a tip and scraper showing path and orientation properties representative of some embodiments of the invention.
FIGS. 22d is a perspective view of a tip and scraper showing path and orientation properties representative of some embodiments of the invention.
FIGS. 22e is a perspective view of a tip and scraper showing path and orientation properties representative of some embodiments of the invention.
FIGS. 22f is a perspective view of a tip and scraper showing path and orientation properties representative of some embodiments of the invention.
FIGS. 22g is a perspective view of a tip and scraper showing path and orientation properties representative of some embodiments of the invention.

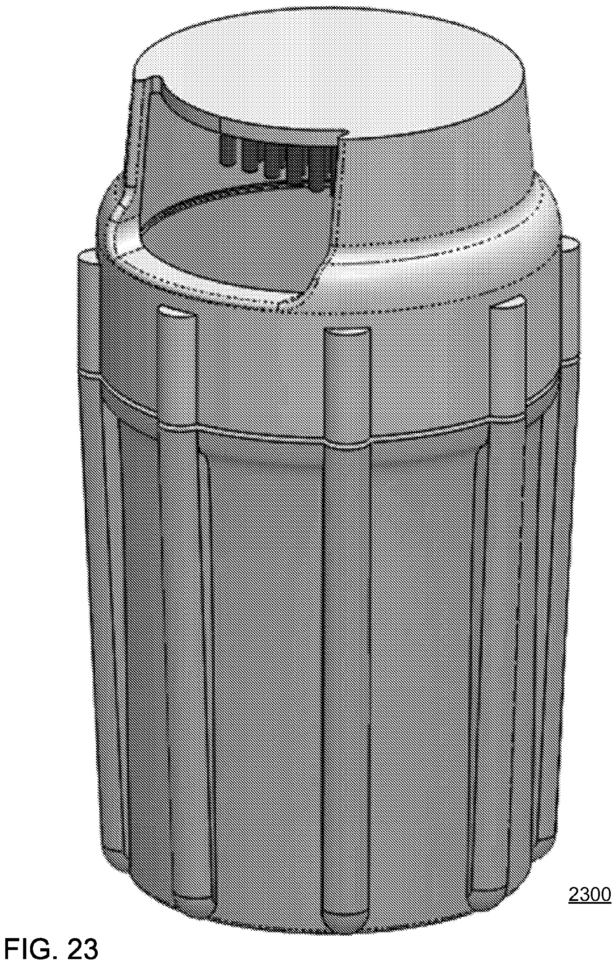

FIG. 23 illustrates a perspective view of a collector in accordance with another embodiment of the invention.

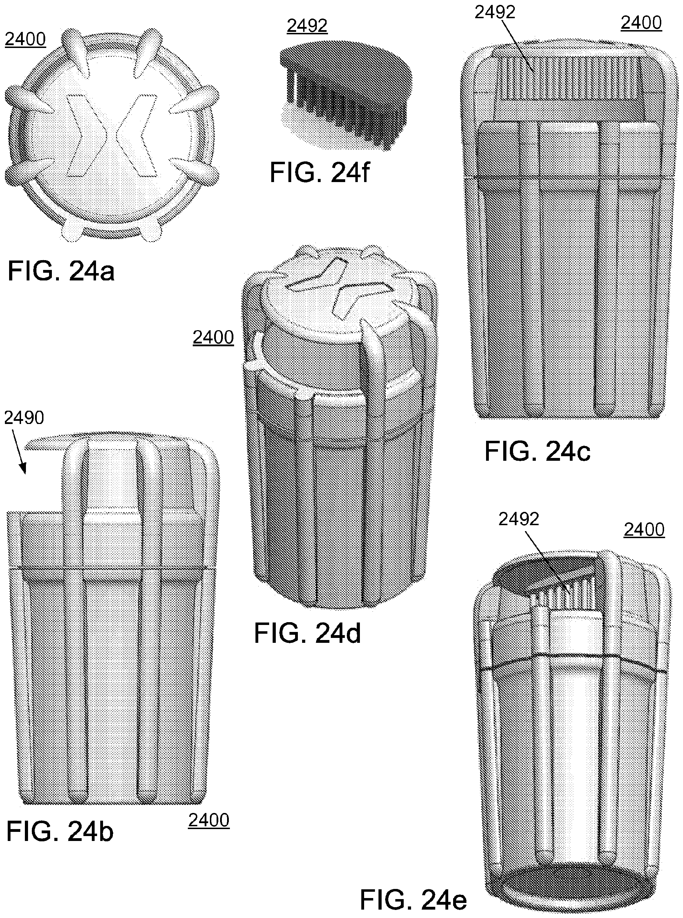

FIG. 24a illustrates a top plan view of a cap of a collector **2400** in accordance with another embodiment of the invention.
FIG. 24b is a side elevational view of the collector **2400.**
FIG. 24c is another side elevational view of the collector **2400.**
FIG. 24d is a top perspective side view of the collector **2400.**
FIG. 24e is a bottom perspective side view of the collector **2400.**
FIG. 24f is a perspective view of the scraper of the collector **2400,** which scraper is located under and attached to the cap, as seen through the opening in the cap in FIGS. 24c and 24e.

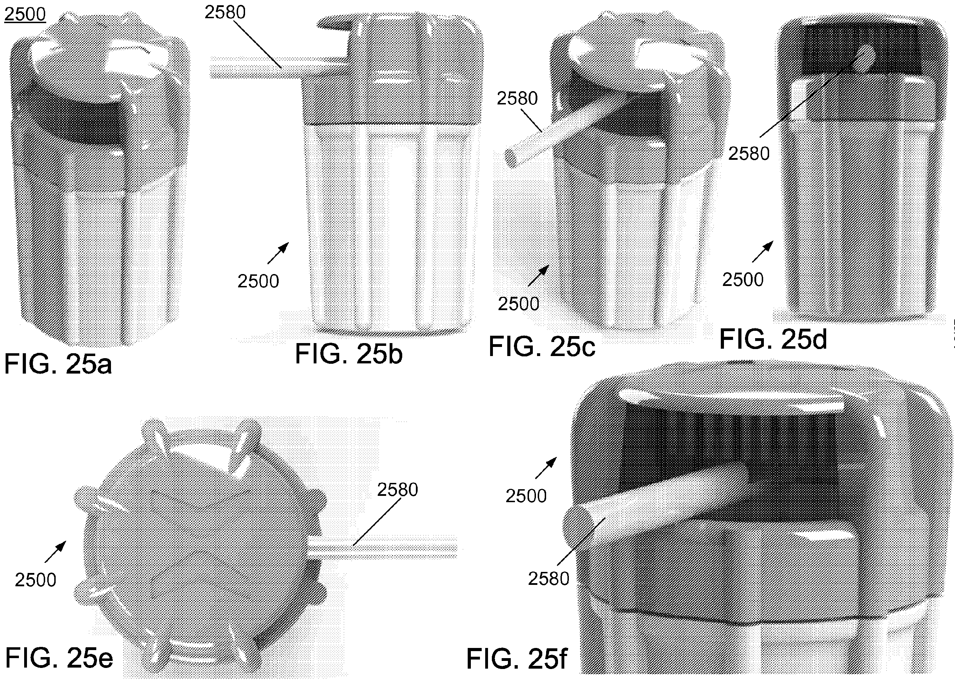

FIG. 25a is a perspective view of a collector in accordance with another embodiment of the invention.
FIG. 25b is a side elevational view of the collector of FIG. 25a, further illustrating a tip of a rongeur inserted into the cap thereof.
FIG. 25c is a perspective view of the collector of FIG. 25a, further illustrating a tip of a rongeur inserted into the cap thereof.

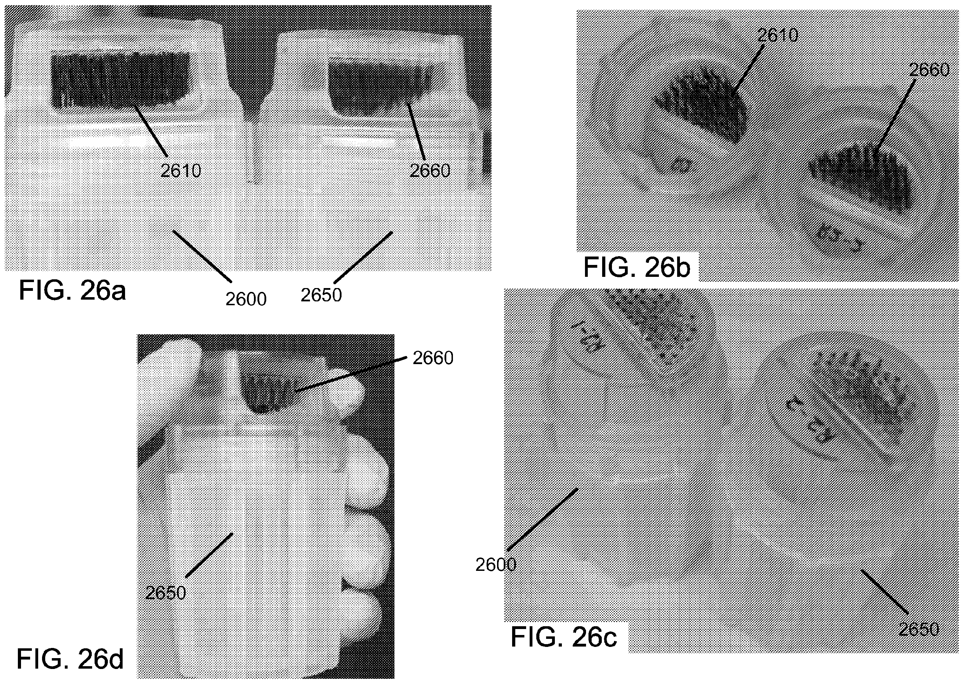

FIG. 26d is another elevational side view of the collector of FIG. 25a, further illustrating a tip of a rongeur inserted into the cap thereof.
FIG. 25e is a top perspective view of the collector of FIG. 25a, further illustrating a tip of a rongeur inserted into the cap thereof.
FIG. 25f is a perspective close-up view of the top of the collector of FIG. 25a with a tip of a rongeur inserted therein, and further illustrating bristles of the brush extending from the underside of the top of the cap downwardly into engagement with the tip of the rongeur.
FIG. 26a is a side-by-side elevational view of the tops of two prototype collectors, each in accordance with a respective embodiment of the invention.
FIG. 26b is a perspective view of the caps of the collectors of FIG. 26a after the caps have been unscrewed from the containers and placed upside down on a surface to expose views of the brushes attached to the underside of the caps.
FIG. 26c is a side-by-side top perspective view of the collectors of FIG. 26a.
FIG. 26d is a side elevational view of one of the collectors of FIG. 26a.

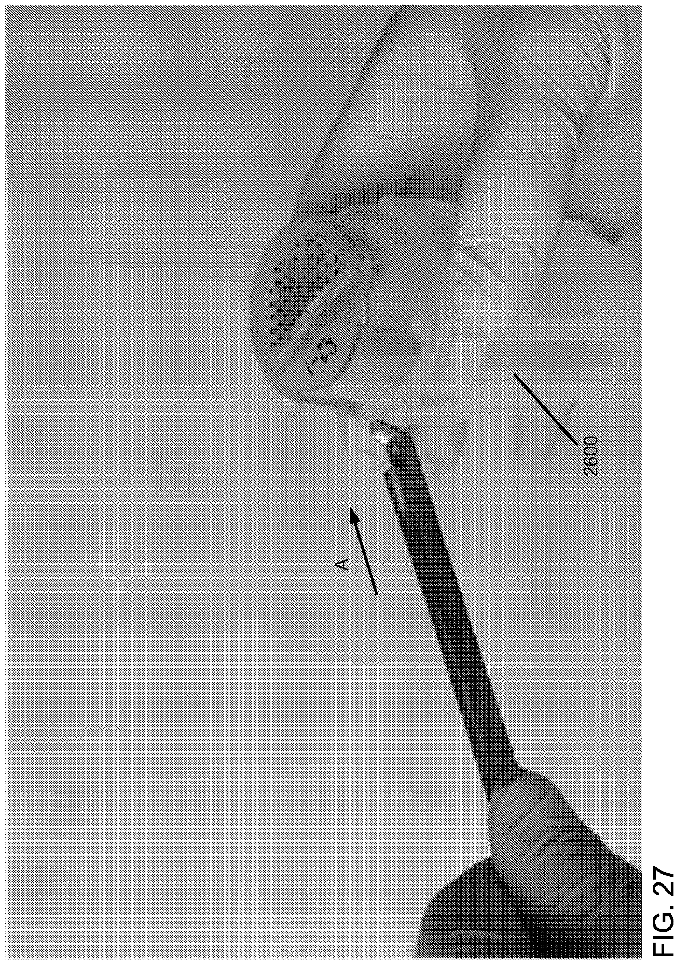

FIG. 27 is a perspective view showing movement of the tip of a rongeur in the direction of arrow **A** so as to extend through a port of a collector in accordance with an embodiment of the invention.

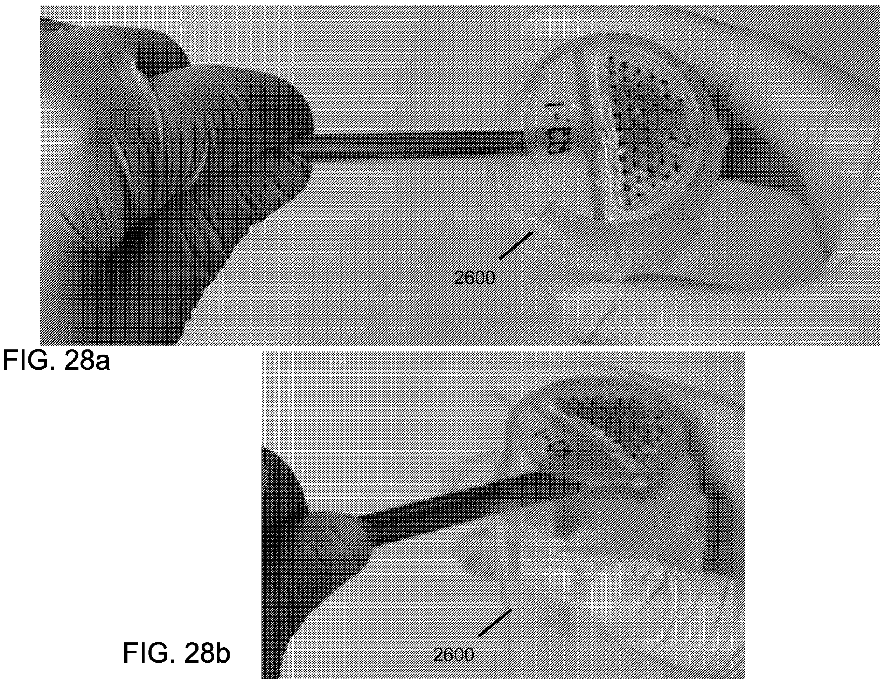

FIG. 28a is another perspective view showing the tip of the rongeur of FIG. 27 received within the cap of the collector of FIG. 27.
FIG. 28b is another perspective view also showing the tip of the rongeur of FIG. 27 received within the cap of the collector of FIG. 27.

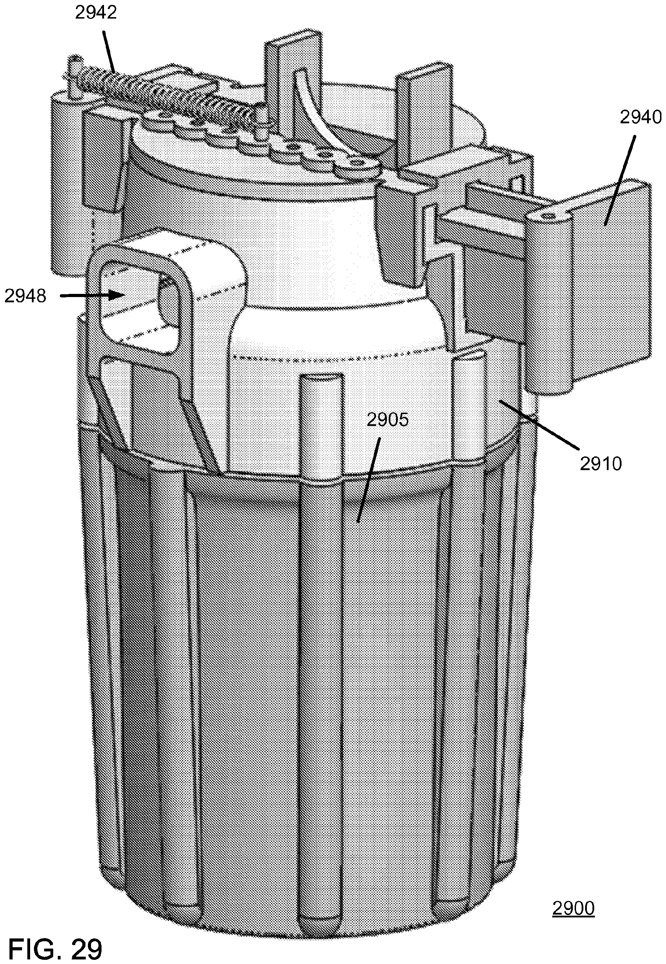

FIG. 29 is a perspective side view of a collector in accordance with yet another embodiment of the invention.

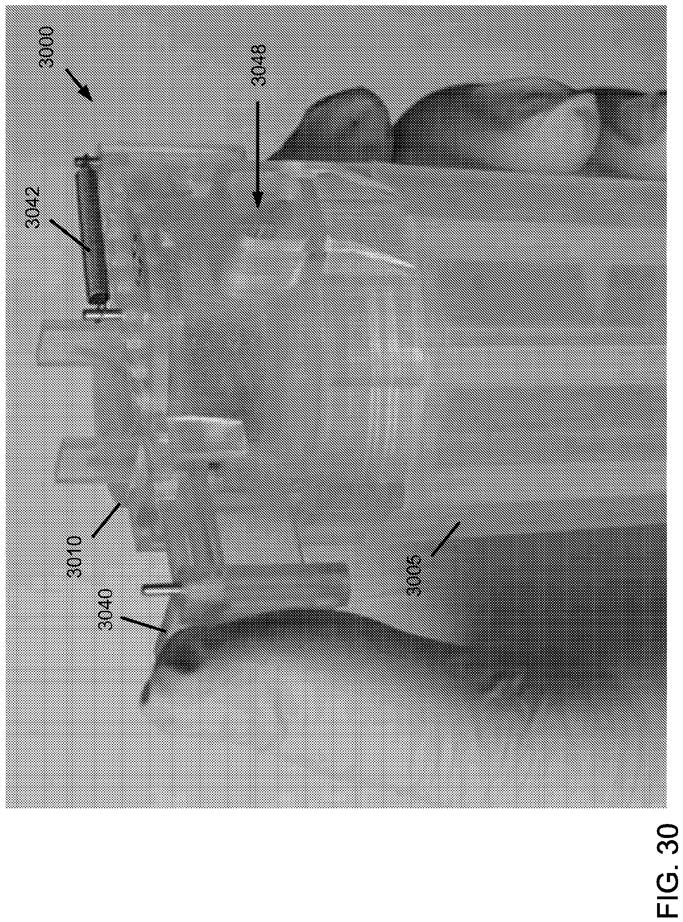

FIG. 30 is a perspective side view of a prototype collector representative of the embodiment of FIG. 29 in accordance with an embodiment of the invention.

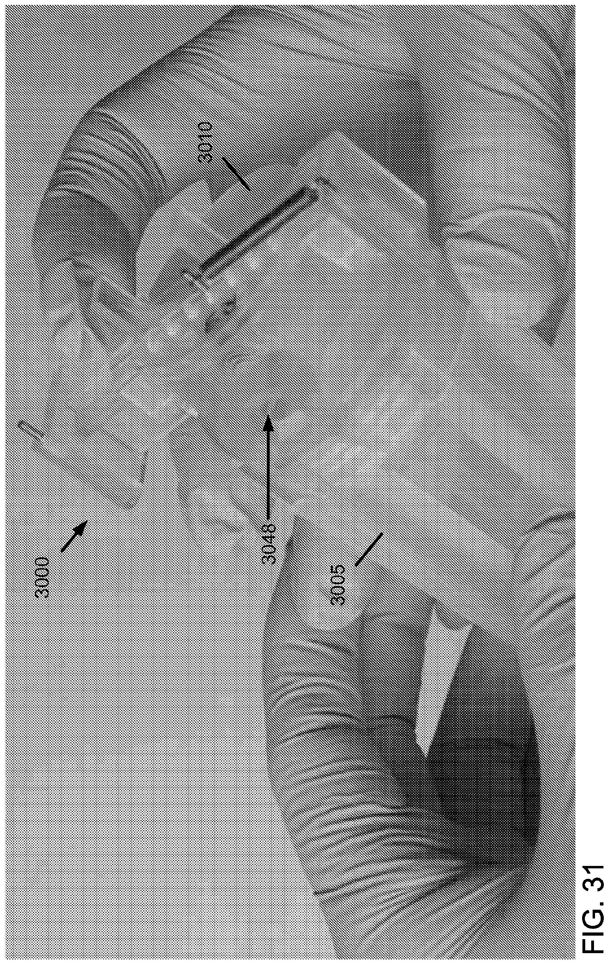

FIG. 31 is another perspective side view of the prototype collector of FIG. 30.

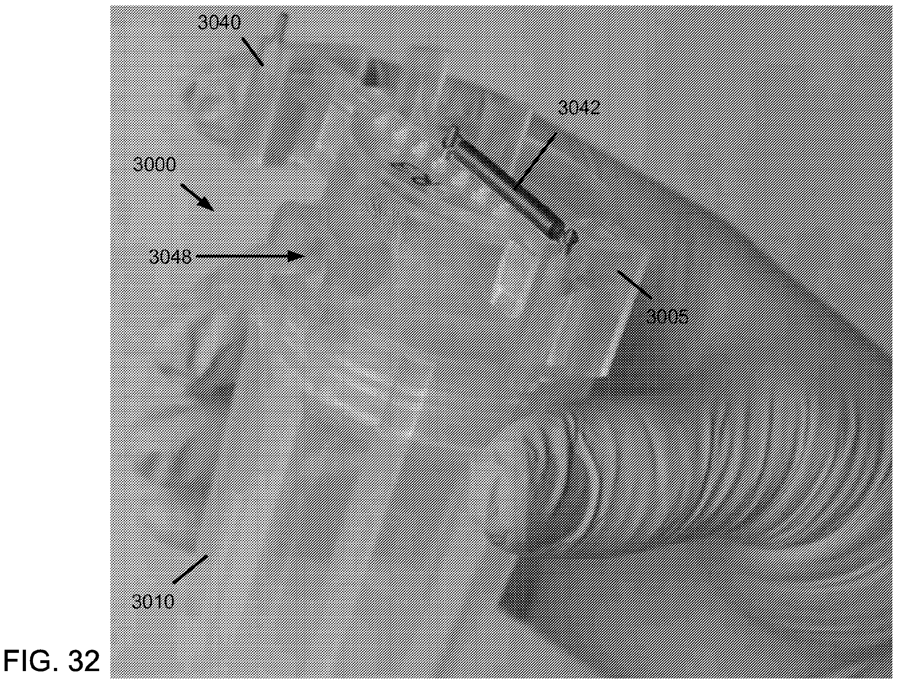

FIG. 32 is another perspective side view of the prototype collector of FIG. 30.

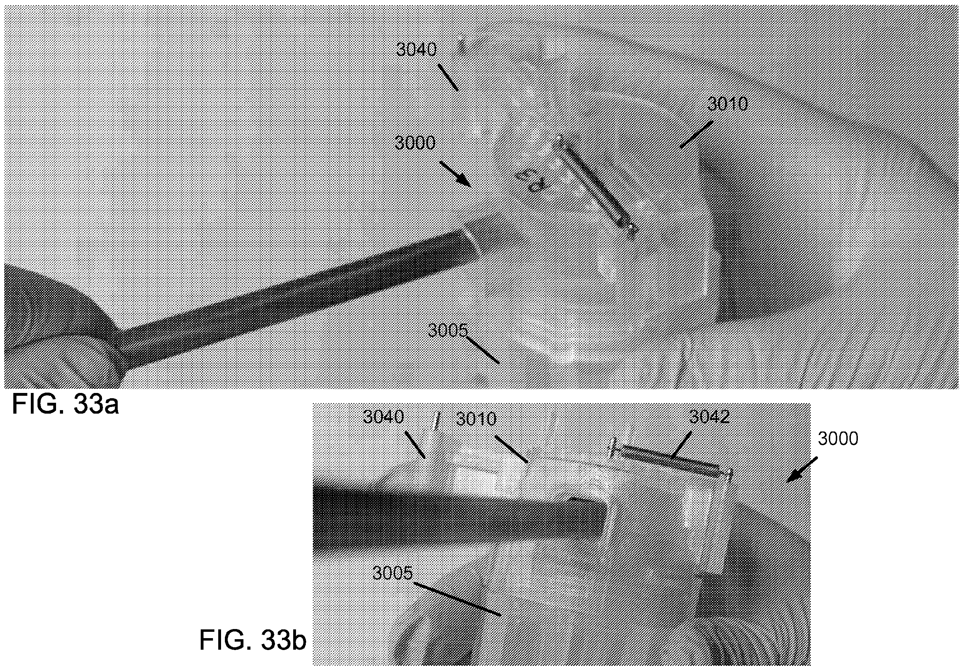

FIG. 33a is a perspective side view of the prototype collector of FIG. 30 with the tip of a rongeur inserted through the port thereof into the collector.
FIG. 33b is another perspective side view of the prototype collector of FIG. 30 with the tip of the rongeur inserted through the port thereof into the collector.

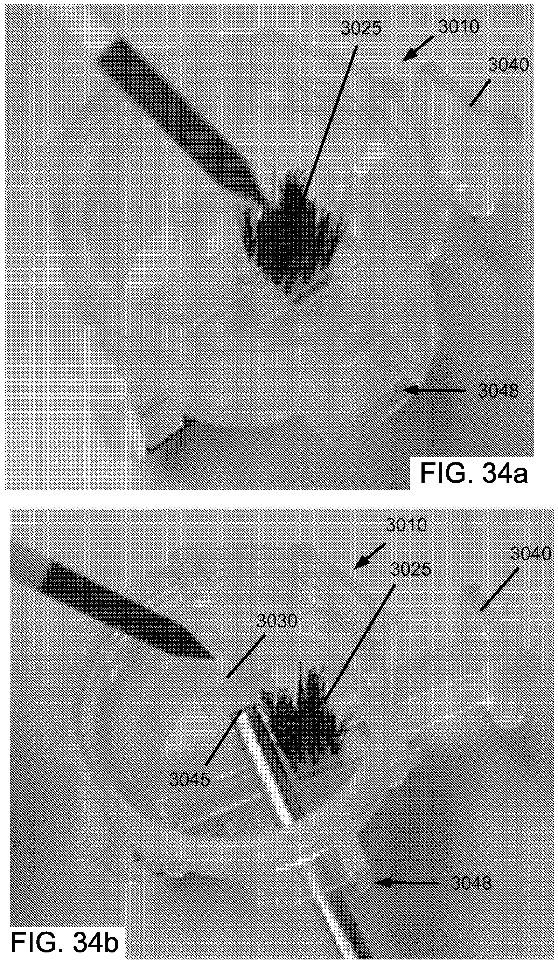

FIG. 34a is a perspective view of the underside of the cap of the prototype collector of FIG. 30, wherein the scraper attached to the plunger perhaps is best seen.
FIG. 34b is a perspective view of the underside of the cap of the prototype collector of FIG. 30, wherein the stop of the cap, against which the tip of the rongeur rests when fully inserted, is perhaps best seen.

### DETAILED DESCRIPTION

As a preliminary matter, it will readily be understood by one having ordinary skill in the relevant art ("Ordinary Artisan") that the present invention has broad utility and application. As should be understood, any embodiment may incorporate only one or a plurality of the above-disclosed aspects of the invention and may further incorporate only one or a plurality of the above-disclosed features. Furthermore, any embodiment discussed and identified as being "preferred" is considered to be part of a best mode contemplated for carrying out the present invention. Other embodiments also may be discussed for additional illustrative purposes in providing a full and enabling disclosure of the present invention. As should be understood, any embodiment may incorporate only one or a plurality of the above-disclosed aspects of the invention and may further incorporate only one or a plurality of the above-disclosed features. Moreover, many embodiments, such as adaptations, variations, modifications, and equivalent arrangements, will be implicitly disclosed by the embodiments described herein.

Accordingly, while the present invention is described herein in detail in relation to one or more embodiments, it is to be understood that this disclosure is illustrative and exemplary of the present invention, and is made merely for the purposes of providing a full and enabling disclosure of the present invention. The detailed disclosure herein of one or more embodiments is not intended, nor is to be construed, to limit the scope of patent protection afforded the present invention in any claim of a patent issuing here from, which scope is to be defined by the claims. It is not intended that the scope of patent protection afforded the present invention be defined by reading into any claim a limitation found herein that does not explicitly appear in the claim itself.

Thus, for example, any sequence(s) and/or temporal order of steps of various processes or methods that are described herein are illustrative and not restrictive. Accordingly, it should be understood that, although steps of various processes or methods may be shown and described as being in a sequence or temporal order, the steps of any such processes or methods are not limited to being carried out in any particular sequence or order, absent an indication otherwise. Indeed, the steps in such processes or methods generally may be carried out in various different sequences and orders while still falling within the scope of the present invention. Accordingly, it is intended that the scope of patent protection afforded the present invention is to be defined by the issued claim(s) rather than the description set forth herein.

Additionally, it is important to note that each term used herein refers to that which the Ordinary Artisan would understand such term to mean based on the contextual use of such term herein. To the extent that the meaning of a term used herein as understood by the Ordinary Artisan based on the contextual use of such term differs in any way from any particular dictionary definition of such term, it is intended that the meaning of the term as understood by the Ordinary Artisan should prevail.

Furthermore, it is important to note that, as used herein, "a" and "an" each generally denotes "at least one," but does not exclude a plurality unless the contextual use dictates otherwise. Thus, reference to "a picnic basket having an apple" describes "a picnic basket having at least one apple" as well as "a picnic basket having apples." In contrast, reference to "a picnic basket having a single apple" describes "a picnic basket having only one apple."

When used herein to join a list of items, "or" denotes "at least one of the items," but does not exclude a plurality of items of the list. Thus, reference to "a picnic basket having cheese or crackers" describes "a picnic basket having cheese without crackers", "a picnic basket having crackers without cheese", and "a picnic basket having both cheese and crackers." Finally, when used herein to join a list of items, "and" denotes "all of the items of the list." Thus, reference to "a picnic basket having cheese and crackers" describes "a picnic basket having cheese, wherein the picnic basket further has crackers," as well as describes "a picnic basket having crackers, wherein the picnic basket further has cheese."

Additionally, as used herein, "cap" denotes "a lid configured to be attached to an object in covering relation to an interior containment space of the object".

As used herein, a "scraper" is a brush, a group of bristles, a protuberance, a barb, or a finger; and is sufficiently rigid so as to generally hold form when not engaged by the distal end of a kerrison rongeur and to generally dislodge bone found in a cutting area of a kerrison rongeur when the finger is moved into or through the cutting area. Preferably, a scraper also is sufficiently flexible and resilient so as to generally deflect and bend to some extent upon abutment by and engagement with the distal end of a kerrison rongeur.

Referring now to the drawings, one or more preferred embodiments of the present invention are next described. The following description of one or more preferred embodiments is merely exemplary in nature and is in no way intended to limit the invention, its implementations, or uses.

### A First Embodiment

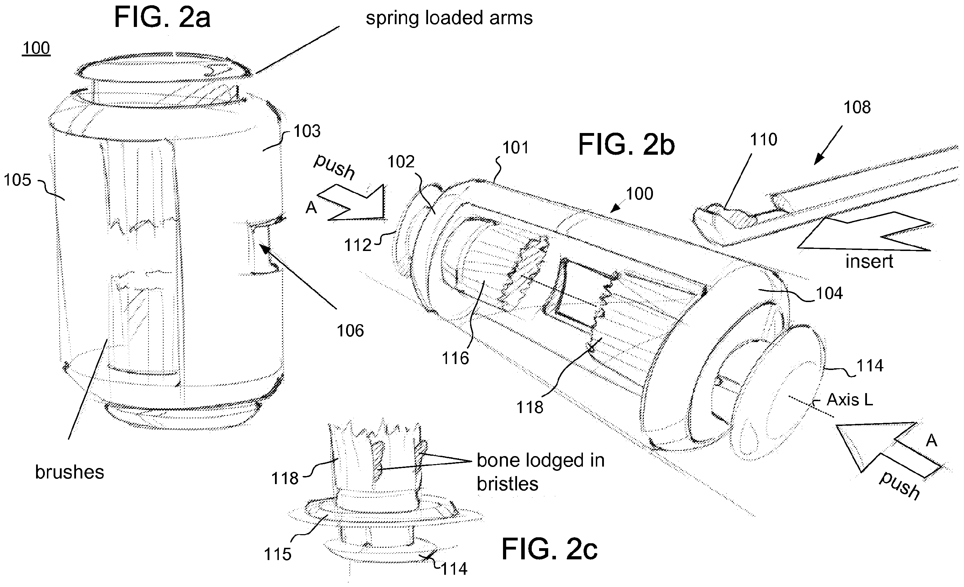

Accordingly, a perspective view of a collector **100** used to collect cut bone from a kerrison rongeur the bone is shown in FIG. 2a. The collector **100** is in accordance with a first embodiment of the present invention and is illustrated in a generally vertical orientation in FIG. 2a. A perspective view of a sequence of steps for using the collector **100** in collecting bone from a kerrison rongeur is represented in FIG. 2b, in which figure the collector **100** is illustrated in a generally horizontal orientation.

As will be appreciated, the collector **100** is hand-held and comprises a container body **101** (shown in FIG. 2a and Fig. 2b to be a generally elongate body or tube that is cylindrical in shape), and having opposite, axially-aligned end caps **102,104.** The container body **101** defines an interior containment space and comprises an opaque wall portion **103** and a transparent wall portion **105.**

In use, the collector **100** is manually held at opposite ends in a generally horizontal position, as shown in FIG. 2b, in which it is positioned to receive the distal end **108** of a kerrison rongeur containing cut bone **110.** The distal end **108** is received through an intake port opening **106** that is located in side wall portion **103** halfway in- between the opposite ends of the collector **100.** In this respect, the collector **100** is held sideways and is said to be side-loaded.

Preferably in use, a scrub nurse, assisting technician, or similar person (hereinafter generally "assistant") moves or loads the collector **100** onto the distal end **108** of the kerrison rongeur when extended from the surgical site by the surgeon, who preferably maintains focus and attention on the surgical site and does not look away for purposes of aligning the distal end **108** with the intake port opening **106** (which alignment is the assistant's responsibility); it will be appreciated that the intake port opening **106** is maneuvered relative to and aligned with the distal end **108** by the assistant.

Upon receipt of the distal end **108** with the cut bone **110** through the opening **106** into the interior of the collector **100,** the assistant depresses (pushes inwardly in direction of arrows A) on spring-loaded, axially-aligned end buttons **112,114** located on the opposite end caps **102,104** of the collector **100,** respectively. In this respect, the buttons preferably are urged or biased by springs (not visible in FIGS. 2a or 2b) in directions opposite to the arrows A in FIG. 2a so as to extend outwardly from the body **101** for grasping and holding of the collector **100** by the assistant during maneuvering of the collector onto the distal end **108** of the kerrison rongeur. This causes opposed interior scrapers **116,118** located in the interior of the collector **100** and connected to the end caps **102,104** to converge onto and cover the distal end **108** of the kerrison rongeur when received within the interior of the collector **100** through the port opening **106.** This action especially when performed multiple times should serve to dislodge the bone **110** from the kerrison rongeur, resulting in the bone **110** either becoming lodged within the scrapers or falling onto an interior wall of the collector **100** defining at least in part the interior containment space. Moreover, the scrapers **116,118** optionally may be rotatable about a longitudinal axis **L** of the container body **101** by respective manual rotation of the end buttons **112,114.** The transparency of the wall portion **105** will enable the assistant to view whether the bone **110** has been collected yet from the kerrison rongeur, and to continue actuating the end buttons **112,114** either until the bone **110** has been collected or a reasonable number of attempts has been tried.

Once collected, the assistant removes (unloads) the collector **100** from the distal end **108** of the kerrison rongeur with the bone **110** remaining within the interior of the collector **100,** either loosely retained therein or lodged within one of the opposed scrapers **116,118.** Preferably the assistant then acknowledges to the surgeon the completion of the collection of the bone from kerrison rongeur, whereupon the surgeon returns the kerrison rongeur to the surgical site for continued cutting. This sequence continues until cutting by the surgeon is completed.

During the procedure, the bone **110** may be kept within the collector **100** until needed or may be removed as desired. The bone **110** preferably is harvested by removing each of the end caps **102,104** from the elongate body **101** of the collector **100.** FIG. 2c is a perspective view of a scraper **118** of an end cap **114** of the collector **100** after bone has been collected from a kerrison rongeur and the end cap **114** has been detached from the body **101.** In this respect, each of the end caps **112,114** preferably is received within an end of the elongate body **101** and retained thereby by a friction fit, for example, between the wall of the body **101** and a rubber or silicone seal **115;** and/or a threaded engagement by screwing (not shown). Bone that is lodged within a scraper **116,118** of an end cap **102,104** can be removed by manually agitating the scraper **116,118** and pulling on the bone, with the scraper **116,118** extending over a sterile cloth, towel, pad, or table surface to catch the falling bone. Similarly, the bone within the body **101** of the collector **100** that is loose may be dumped onto a sterile cloth, towel, or pad, or directly onto a sterile surface of a table. The transparent portion **105** of the body **101** further may comprise a hinged or removable panel, by which the interior of the body **101** may be accessed for manual removal of any bone that may become stuck within the interior containment space of the body **101.** The harvested bone then can be used during the procedure, such as for example, in an autograft procedure.

The materials from which the components of the collector are made may be any desired, suitable material for use in the foregoing procedure. For instance, a scraper may comprise metal or other rigid or semi-rigid material that is effective in dislodging bone from the distal end of the kerrison rongeur. It is further contemplated that one or more of the materials may be molded from, for example, an inert plastic material. Furthermore, the materials preferably are lightweight such that the collector is readily manipulated by hand. Furthermore, the collector may be disposable, in that the collector may be used during a single medical procedure for a patient and then discarded in accordance with applicable HAZMAT protocols. Alternatively, one or more components of the collector (including all of the components) are designed to and are able to be sterilized for reuse with another patient during another procedure.

### A Second Embodiment

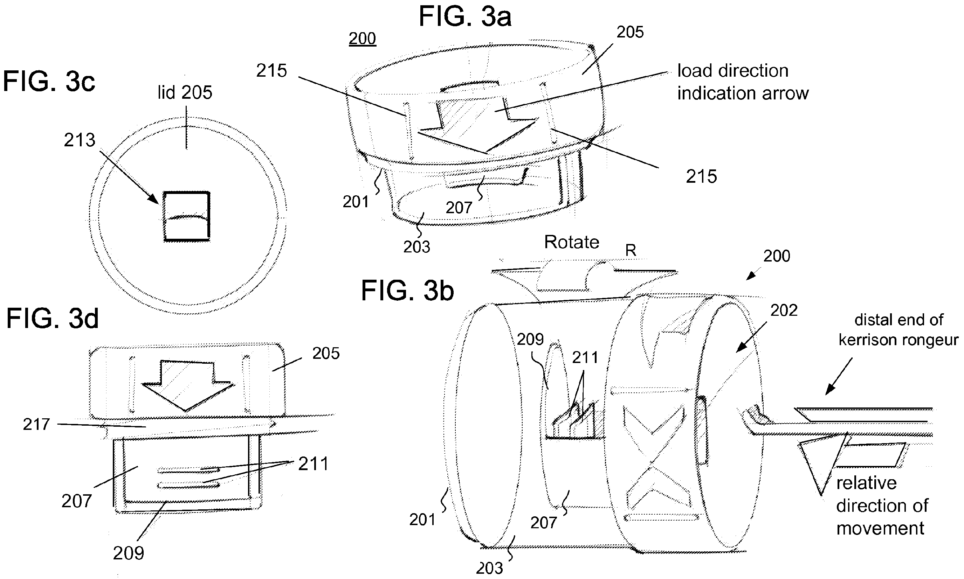

FIG. 3a is a perspective view of a collector **200** used to collect cut bone from a kerrison rongeur in accordance with a second embodiment of the present invention. The collector **200** is illustrated in a generally vertical orientation in FIG. 3a. A perspective view of the collector **200** representing a sequence of steps for using the collector **200** to collect bone from a kerrison rongeur is shown in FIG. 3b, wherein the collector **200** is illustrated in a generally horizontal orientation.

The collector **200** is hand-held and comprises a container body **201,** shown to be generally cylindrical in shape. The container body **201** defines an interior containment space and includes a transparent sidewall portion **203** through which the interior of the container body **201** is visible.

The collector **200** further comprises a cap **205** that is attached to the container body **201.** FIG. 3c is a top plan view of the cap **205** of the collector **200,** and FIG. 3d is a side elevational view of the cap **205** of the collector **200.**

The top of the cap **205** includes an intake port opening **213** centrally located in the top of the cap **205,** perhaps as best shown in FIG. 3c. The top of the cap **205** includes a funnel-shaped surface **202** that is radially inclined toward the opening **213,** as perhaps best shown in FIG. 3b. The cap **205** further includes a platform **207** that extends into the interior of the container body **201,** as perhaps best shown in FIG. 3b. The platform **207** includes a stop **209** and scrapers **211.** The scrapers **211** two of which are shown in the figures, but any number of which may be included extend from the platform and are flexible and resilient, preferably sufficiently rigid or semi-rigid so as to hold form when not engaged by the distal end of a kerrison rongeur and deflecting and bending upon abutment by and engagement with the distal end of a kerrison rongeur. The scrapers **211** may be made from metal or other material, and it is contemplated that a scraper may be made of polypropylene.

It will be appreciated that the funnel-shaped top surface **202** of the cap **205** is designed to direct a distal end of a kerrison rongeur toward the opening **213** upon abutment therewith, and the stop **209** is intended to abut and physically preclude further advancement of the distal tip of the kerrison rongeur when received through the opening **213.**

The cap **205** is freely rotatable relative to the container body **201,** as indicated by the arrow R in FIG. 3b. Rotation or twisting of the cap **205** on the container body **201** is facilitated by external ribs **215** on the side of the cap **205** for manual grasping. Rotation of the cap **205** does not detach the cap **205** from the container body **201,** which preferably is received and held within a mouth of the container body **201** in a frictional fit between the container body and a seal **217** of the cap **205.** The cap **205** preferably is removable by pulling of the cap **205** off of the container body **201** with a reasonable amount of effort.

The stop **209** is located at a spacing relative to the opening **213** such that, upon receipt of the distal end of the kerrison rongeur and abutment thereof with the stop **209,** the scrapers **211** extend over and cover the distal end of the kerrison rongeur proximate the cutting area in which cut bone would be found. Furthermore, rotation of the cap results in movement of the scrapers **211** around, about and through the cutting area of the distal end of the kerrison rongeur so as to dislodge and remove for collection any cut bone carried therein.

In this respect, the collector **200** is held horizontally generally as shown in FIG. 3b.

Preferably in use, an assistant moves or loads the collector **200** onto the distal end of a kerrison rongeur when extended from the surgical site by the surgeon, who preferably maintains focus and attention on the surgical site and does not look away for purposes of aligning the distal end with the intake port opening **213,** which is generally located in the center of the cap **205.** Alignment is the assistant's responsibility, and it will be appreciated that the intake port opening **213** is maneuvered relative to and aligned with the distal end of the kerrison rongeur by the assistant. The funnel-shaped surface **202** facilitates the alignment with and receipt of the distal tip within the opening **213.**

Upon receipt of the distal end of the kerrison rongeur into the interior of the collector **200** and its abutment with the stop **209,** the assistant twists (rotates in direction of arrow R) the cap **205** relative to the container body **201.** This causes the scrapers **211** to engage and remove any cut bone carried by the kerrison rongeur, resulting in the bone either falling onto an interior wall of the container body **201** or becoming lodged within a scraper. Moreover, the transparency of the container body **201** will enable the assistant to view whether the bone has been collected yet from the kerrison rongeur, and to continue twisting the cap **205** on and relative to the container body **201** either until the bone has been collected or a reasonable number of rotations has occurred.

Once collected from the kerrison rongeur, the assistant removes (unloads) the collector **200** from the distal end of the kerrison rongeur with the bone remaining within the interior of the collector **200,** either loosely retained therein or lodged within a scraper. Preferably the assistant then acknowledges to the surgeon the completion of the collection of the bone from kerrison rongeur, whereupon the surgeon returns the kerrison rongeur to the surgical site for continued cutting. This sequence continues until cutting by the surgeon is completed.

During the procedure, the bone may be kept within the collector **200** until needed or may be removed as desired. The bone preferably is harvested by removing the cap **205** from the container body **201.** Bone that is lodged within a scraper can be removed by manually agitating the scraper and pulling on the bone so that the bone falls onto a sterile cloth, towel, pad, or table surface. The bone within the container body **201** that is loose may be dumped onto the sterile cloth, towel, or pad, or directly onto the sterile surface of the table. Bone within the container body **201** that is not loose may be manually removed. The harvested bone then can be used during the procedure, such as for example, in an autograft procedure.

The materials from which the components of the collector are made may be any desired, suitable material for use in the foregoing procedure. For instance, a scraper may comprise metal or other rigid or semi-rigid material that is effective in dislodging bone from the distal end of the kerrison rongeur. It is further contemplated that one or more of the materials may be molded from, for example, an inert plastic material. Furthermore, the materials preferably are lightweight such that the collector is readily manipulated by hand. Furthermore, the collector may be disposable, in that the collector may be used during a single medical procedure for a patient and then discarded. Alternatively, one or more components of the collector (including all of the components) are designed to and are able to be sterilized for reuse with another patient during another procedure.

### A Third Embodiment

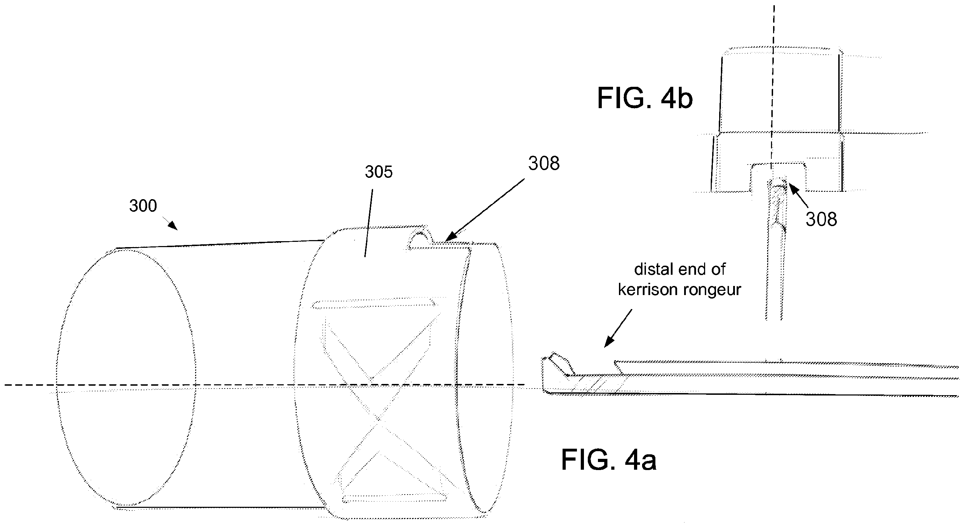

FIG. 4a is a perspective view illustrating a sequence of steps for using another collector **300** in accordance with a third embodiment of the present invention, wherein the collector **300** is illustrated in a generally horizontal orientation receiving the distal end of a kerrison rongeur. FIG. 4b is a top plan view of the collector **300** and distal tip of the kerrison rongeur of FIG. 4a. The collector **300** and use thereof is similar to collector **200** and its use, with the exception that a cap **305** of the collector includes a cut out **308** in the side thereof by which an assistant may better visualize alignment with and receipt of the distal end of a kerrison rongeur within the intake port opening in the cap **305,** and with the further exception that the cap further does not include a funnel-shaped top surface.

### A Fourth Embodiment

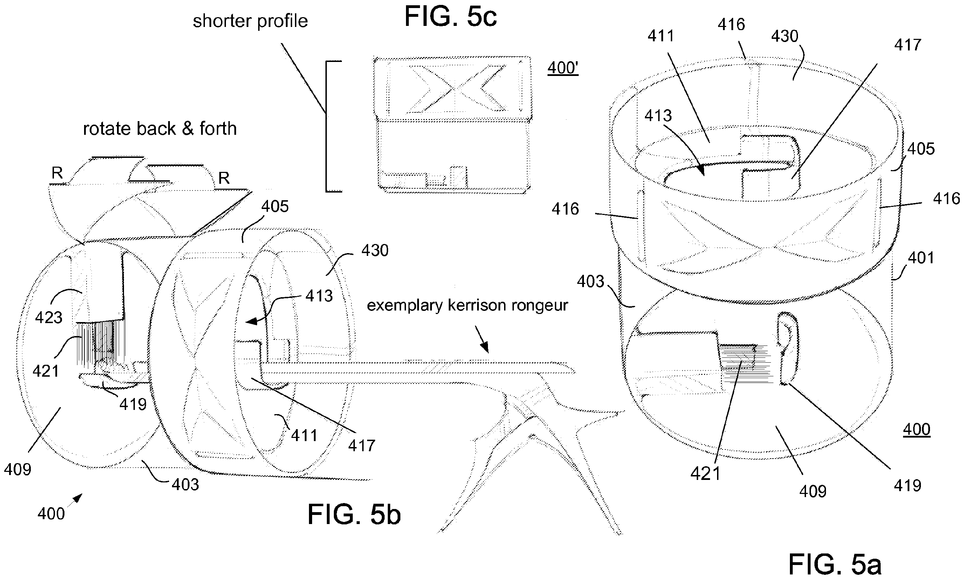

FIG. 5a a perspective view of a collector **400** used to collect cut bone from a kerrison rongeur for harvesting in accordance with another embodiment of the present invention, wherein the collector **400** is illustrated in a generally vertical orientation. FIG. 5b is a perspective view representative of a sequence of steps for using the collector **400** in collecting bone from a kerrison rongeur, wherein the collector **400** is illustrated in a generally horizontal orientation with the distal end of the kerrison rongeur being received within the collector **400.**

The collector **400** is hand-held and comprises a container body **401,** shown to be generally cylindrical in shape. The container body **401** defines an interior containment space and includes a transparent sidewall portion **403** through which the interior of the container body **401** is visible. The collector **400** further comprises a cap **405** that is attached to the container body **401.** The cap **405** includes an interior ledge **411** that defines an intake port opening **413** through which the distal end of a kerrison rongeur is received. The cap **405** further includes a guide member **417** that projects from the ledge **411** and that is shaped and contoured for receiving and guiding therein a portion of the distal end of the kerrison rongeur. The guide member **417** preferably includes a U-shape configuration, as shown. An saddle member **419** extends from a bottom **409** of the container body **401** and, in conjunction with the guide member **417,** assists in aligning the distal end of the kerrison rongeur for collection of bone, as shown in FIG. 5b. The saddle member **419** preferably includes a C-shape configuration, as shown.

A scraper **421** is provided proximate the bottom **409** of the container body **401** and extends toward the saddle member **419** into the cutting area of the distal end of a kerrison rongeur when aligned by the saddle member **419.** The scraper **421** is secured to a base **423** that extends from the side wall **403** of the container body **401** in abutment with the bottom **409** of the container body **401.** Further in this regard, the bottom **409** serves as a stop for the distal end of the kerrison rongeur.

The cap **405** is freely rotatable relative to the container body **401,** as indicated by the arrows R in FIG. 5b. Rotation or twisting of the cap **405** on the container body **401** is facilitated by external ribs **416** on the side of the cap **405** for manual grasping. Rotation of the cap **405** does not detach the cap **405** from the container body **401,** which preferably is received and held within a mouth of the container body **401** in a frictional fit between the container body and a seal (nOw shown, but similar to seal **217** found in collector **200**). The cap **405** preferably is removable by pulling of the cap **405** off of the container body **401** with a reasonable amount of effort.

The scraper **421** is located at a spacing relative to the saddle member **419** such that, upon receipt of the distal end of the kerrison rongeur and abutment thereof with the bottom **409** serving as a stop, the scraper **411** extends over and covers the distal end of the kerrison rongeur proximate the cutting area thereof in which cut bone would be found. Furthermore, rotation of the container body **401** relative to the cap **405** results in movement of the scraper **421** around, about and through the cutting area of the distal end of the kerrison rongeur so as to dislodge and remove for collection any cut bone carried therein. It will further be appreciated that the saddle member **419** also rotates with the container body **401,** whereas the guide member **417** is an integral molded part of--and remains fixed relative to the cap **405.**

It will further be noted that the cap **405** defines an inner surface **430** that, when the collector is positioned for receiving the distal end of the kerrison rongeur, as shown in FIG. 5b, the inner surface **430** will form a flange that catches bone that may fall from the kerrison rongeur while attempting to receiving the distal end of the kerrison rongeur within the guide member **417** and through the intake opening **413.**

Preferably in use, an assistant moves or loads the collector **400** onto the distal end of a kerrison rongeur when extended from the surgical site by the surgeon, who preferably maintains focus and attention on the surgical site and does not look away for purposes of aligning the distal end with the intake port opening **413,** which is generally located in the center of the cap **405.** Alignment is the assistant's responsibility, and it will be appreciated that the intake port opening **413** is maneuvered relative to and aligned with the distal end of the kerrison rongeur by the assistant. The guide member **417** further aids the assistant in positioning of the distal end of the kerrison rongeur within the saddle member **419** after being inserted through the opening **413.**

Upon receipt of the distal end of the kerrison rongeur into the interior of the collector **400** and its abutment with the bottom **409** serving as a stop, the assistant twists (rotates back and forth in the directions of arrows R) the container body **401** relative to the cap **405.** This causes the scraper **421** to engage and remove any cut bone carried by the kerrison rongeur, resulting in the bone either falling onto an interior wall of the container body **401** or becoming lodged within the bristles of the scraper **421.** Moreover, the transparency of the side wall **403** container body **401** enables the assistant to view whether the bone has been collected yet from the kerrison rongeur, and to continue twisting the container body **401** relative to the cap **405** back and forth either until the bone has been collected or a reasonable number of attempts have been made by the assistant.

Once collected from the kerrison rongeur, the assistant removes (unloads) the collector **400** from the distal end of the kerrison rongeur with the bone remaining within the interior of the collector **400,** either loosely retained therein or lodged within the bristles. Preferably the assistant then acknowledges to the surgeon the completion of the collection of the bone from kerrison rongeur, whereupon the surgeon returns the kerrison rongeur to the surgical site for continued cutting without taking focus away from the surgical site. This sequence continues until cutting by the surgeon is completed.

During the procedure, the bone may be kept within the collector **400** until needed or may be removed as desired. The bone preferably is harvested by removing the cap **405** from the container body **401.** Bone that is lodged within a scraper can be removed by manually agitating the bristles and pulling on the bone so that the bone falls onto a sterile cloth, towel, pad, or table surface. The bone within the container body **401** that is loose may be dumped onto the sterile cloth, towel, or pad, or directly onto the sterile surface of the table. Bone within the container body **401** that is not loose may be manually removed by hand or with an instrument. The harvested bone then can be used during the procedure, such as for example, in an autograft procedure.

The materials from which the components of the collector are made may be any desired, suitable material for use in the foregoing procedure. For instance, a scraper may comprise metal or other rigid or semi-rigid material that is effective in dislodging bone from the distal end of the kerrison rongeur. It is further contemplated that one or more of the materials may be molded from, for example, an inert plastic material. Furthermore, the materials preferably are lightweight such that the collector is readily manipulated by hand. Furthermore, the collector may be disposable, in that the collector may be used during a single medical procedure for a patient and then discarded. Alternatively, one or more components of the collector (including all of the components) are designed to and are able to be sterilized for reuse with another patient during another procedure.

FIG. 5c is a side elevational view of an alternative collector **400'** generally similar to the collector **400,** but in which the height of the collector **400'** is less than the height of the collector **400** and, thus, the collector **400'** has a smaller profile.

### A Fifth Embodiment

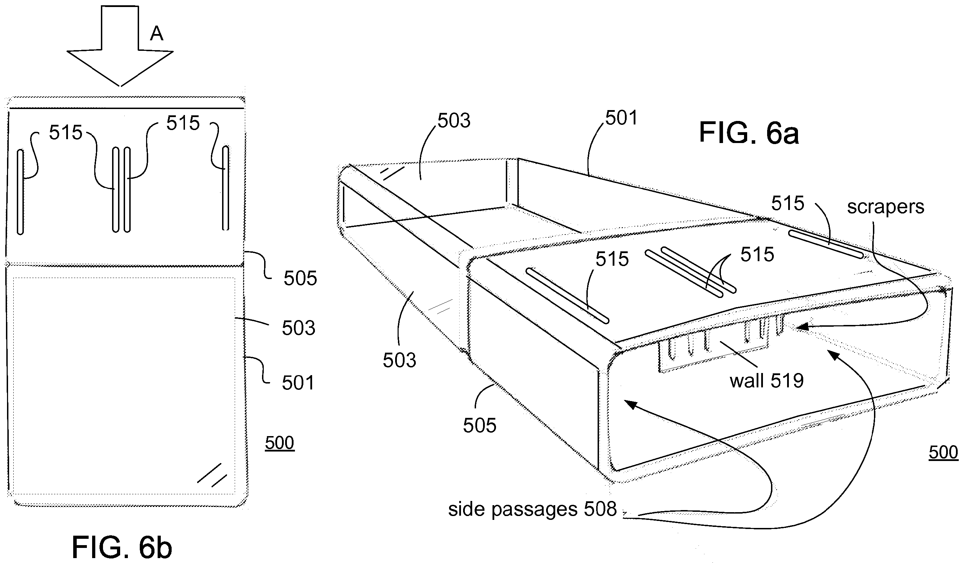

FIG. 6a is a perspective view of a collector **500** used to collect cut bone from a kerrison rongeur for harvesting in accordance with another embodiment of the present invention, wherein the collector **500** is illustrated in a generally horizontal orientation. FIG. 6b is a side elevational view of the collector **500.**

The collector **500** includes a container body **501** that is box-shaped with generally rectangular sides and a cap **505** with generally rectangular sides. The container body **501** defines an interior containment space and preferably includes transparent side walls **503.** The container body **501** is utilized to store collected bone. The cap **505** includes a stop comprising a wall **519** that extends between opposed interior side surfaces of the cap **505** and divides passage through the cap to the interior of the container body **501** into two side passages **508** around opposite sides of the wall **519.** Scrapers **521** are located in front of the wall and extend from one of the interior side surfaces of the cap **505** from to which the wall **519** connects.

Preferably in use, an assistant moves or loads the collector **500** onto the distal end of a kerrison rongeur when extended from the surgical site by the surgeon, who preferably maintains focus and attention on the surgical site and does not look away for purposes of aligning the distal end of the kerrison rongeur with the opening in the cap **505.** Alignment is the assistant's responsibility, and it will be appreciated that the collector **500** is maneuvered relative to and aligned with the distal end of the kerrison rongeur by the assistant such that the distal end of the kerrison rongeur, when received within the cap **505,** will come into abutment with the wall **519** and cease further advancement within the cap **505** toward the container body **501.** The spacing of the wall **519** to the scrapers **521** is such that the scrapers will pass through the cutting area of the distal end of the kerrison rongeur when the collector **500** is moved back and forth in lateral directions relative to the longitudinal extent of the kerrison rongeur. Furthermore, the wall **519** may be maintained in abutment with the tip of the kerrison rongeur during such back and forth movement for proper alignment of the scarpers **521** relative to the cutting area of the kerrison rongeur, thereby facilitating dislodgment of cut bone found in the cutting area by the scrapers **521.** Such back and forth movement is facilitated by longitudinally protruding ribs **515** on the cap **505,** which enable better grasping by hand of the cap **505.** Additionally, one or more ribs **515** such as the middle pair of ribs shown in FIGS. 6a and 6b, for example may be used for sighting by the assistant during loading of the distal end of the kerrison rongeur, as represented by the direction and alignment of arrow A relative to the middle pair of ribs seen in FIG. 6b.

Once collected from the kerrison rongeur, the assistant removes (unloads) the collector **500** from the distal end of the kerrison rongeur with the bone remaining within the interior of the cap **505,** either loosely retained therein or lodged within the bristles. Tilting of the collector **500** to a vertical orientation and light shaking of the collector **500** should cause any loose bone collected from the kerrison rongeur to fall through one of the side passages into the container body **501** where the bone yield can be viewed through the transparent walls **503.**

Preferably the assistant acknowledges to the surgeon the completion of the collection of the bone from kerrison rongeur, whereupon the surgeon returns the kerrison rongeur to the surgical site for continued cutting without taking focus away from the surgical site. This sequence continues until cutting by the surgeon is completed.

During the procedure, the bone may be kept within the container body 5b01 of the collector **500** until needed or may be removed as desired. The bone preferably is harvested by removing the cap **505** from the container body **501.** Bone that is lodged within any bristles of the scrapers **521** can be removed by manually agitating the bristles and pulling on the bone so that the bone falls onto a sterile cloth, towel, pad, or table surface. The bone within the container body **501** that is loose may be dumped onto the sterile cloth, towel, or pad, or directly onto the sterile surface of the table. Bone within the container body **501** that is not loose may be manually removed by hand or with an instrument. The harvested bone then can be used during the procedure, such as for example, in an autograft procedure.

The materials from which the components of the collector are made may be any desired, suitable material for use in the foregoing procedure. For instance, a scraper may comprise metal or other rigid or semi-rigid material that is effective in dislodging bone from the distal end of the kerrison rongeur. It is further contemplated that one or more of the materials may be molded from, for example, an inert plastic material. Furthermore, the materials preferably are lightweight such that the collector is readily manipulated by hand. Furthermore, the collector may be disposable, in that the collector may be used during a single medical procedure for a patient and then discarded. Alternatively, one or more components of the collector (including all of the components) are designed to and are able to be sterilized for reuse with another patient during another procedure.

### A Sixth Embodiment

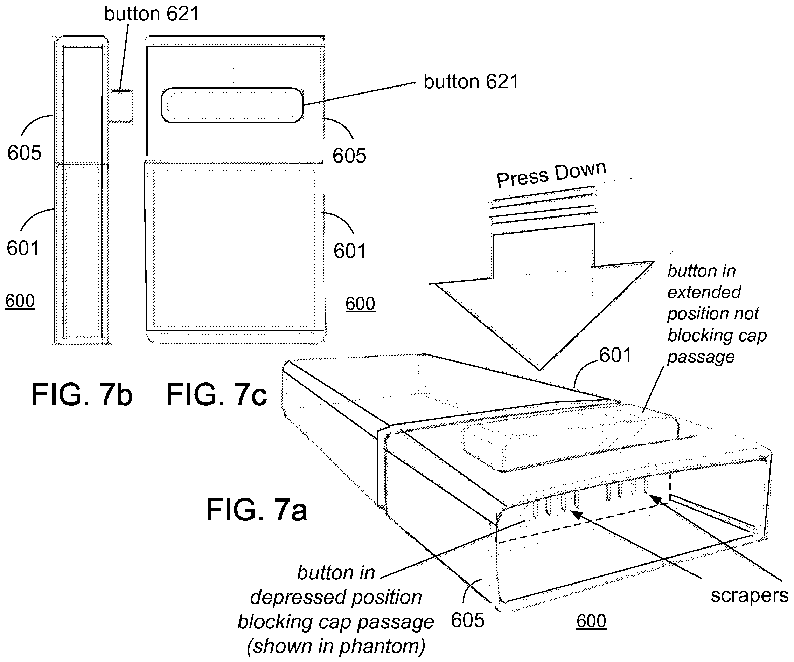

FIG. 7a is a perspective view of a collector **600** used to collect cut bone from a kerrison rongeur in accordance with another embodiment of the present invention, wherein the collector **600** is illustrated in a generally horizontal orientation. Each of FIGS. 7b,7c is a respective side elevational view of the collector **600.** The collector **600** is similar in construction and use as the collector **500** discussed above with reference to FIGS. 6a and 6b. A primary difference over the collector **500,** however, is that the wall **521** is replaced with a depressible button **621.** The button **621** preferably is spring-loaded and biased into an extended position, shown in FIG. 7a in solid line and additionally shown in FIG. 7b, in which extended position passage through the cap **605** to an interior containment space of the container body **601** is not blocked. Upon being fully depressed, the button extends into and blocks passage through the cap **605,** as shown in phantom in FIG. 7a. When in the depressed position, the button serves as a stop in same manner as wall **519** in collector **500,** described above.

### A Seventh Embodiment

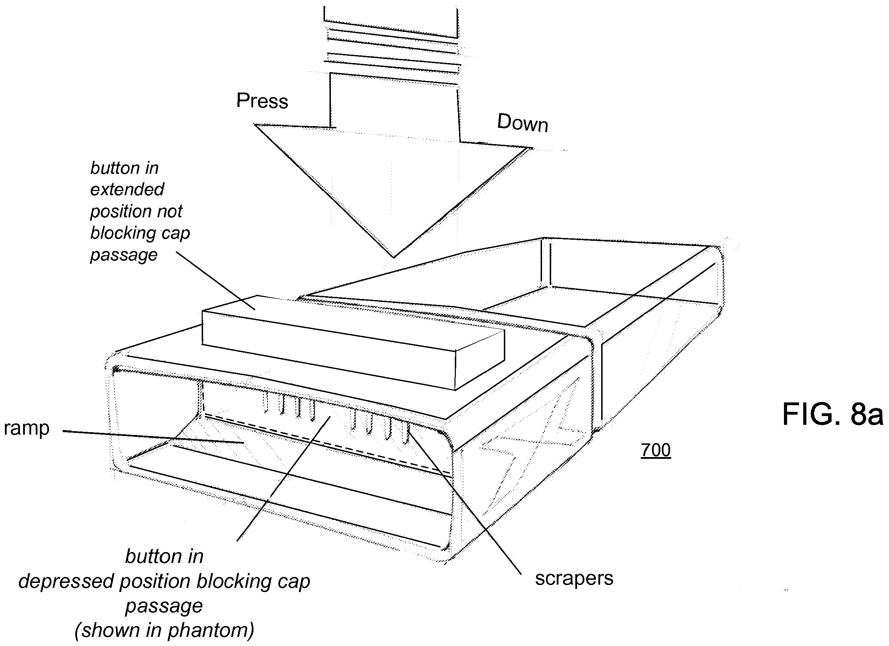

FIG. 8a is a perspective view of a collector **700** used to collect cut bone from a kerrison rongeur for harvesting in accordance with another embodiment of the present invention, wherein the collector **700** is illustrated in a generally horizontal orientation. The collector **700** is similar in construction and use as the collector **600** discussed above with reference to FIG. 7a, FIG. 7b, and FIG. 7c. A primary difference over the collector **600,** however, is that the cap includes a ramp **704** that assists in guiding the tip of the end portion of a kerrison rongeur into proper position within the cap. The height of the passage is also less than that of the height of the passage through cap **605,** and the length of travel when depressing the button is consequently less as well.

### An Eighth Embodiment

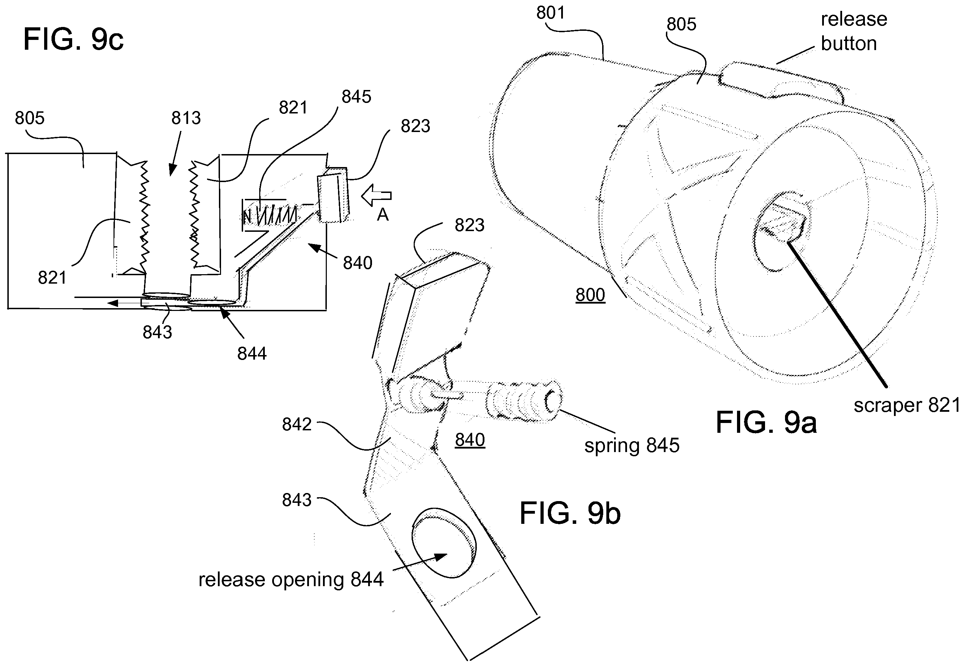

FIG. 9a is a perspective view of a collector **800** used to collect cut bone from a kerrison rongeur for harvesting in accordance with another embodiment of the present invention, wherein the collector **800** is illustrated in a generally horizontal orientation. FIG. 9b is a perspective view of part of a release mechanism **840** of the collector **800,** and FIG. 9c is a schematic illustration representing the arrangement and operation of the release mechanism **840** relative to the scrapers **821** and cap **805** of the collector **800.**

The collector **800** includes a release mechanism **840** having a button **823** connected by an arm **842** to a wall **843.** The wall **843** defines a release opening **844** therein. The release mechanism **840** is contained within the cap **805** and includes a spring **845** that biases the release mechanism **840** into a closed configuration, wherein bone collected within intake port **813** using scrapers **821** is blocked from access to an interior containment space of the container body **801.** When the button **823** is depressed against the biasing of the spring **845** in the direction of arrow A, the wall **843** moves (slides) so as to align the release opening **844** with the intake port opening **813** thereby permitting the collected bone to pass to the interior of the container body **801.** When in the closed configuration, the wall **843** serves as a stop for the distal end of a kerrison rongeur, in which position the scrapers **821** pass through the cutting area when the cap **805** is rotated about its longitudinal axis relative to the kerrison rongeur.

### Additional Embodiments Utilizing Suction

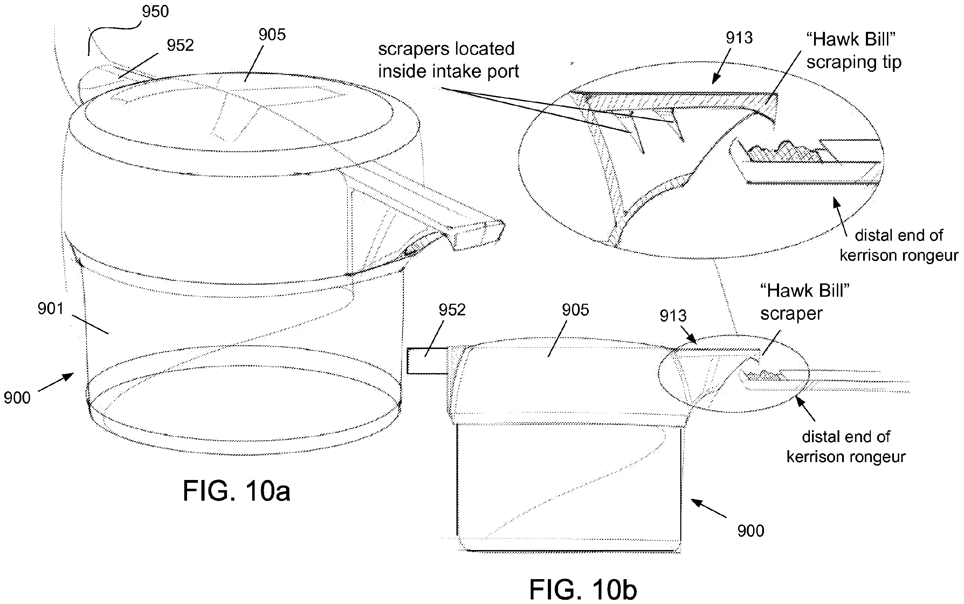

Additional collectors used to collect cut bone from a kerrison rongeur for harvesting in accordance with still yet more embodiments of the present invention embodiments are disclosed with reference to FIGS. 10a through 19c. Each of these additional embodiments utilizes suction and includes a suction port for attachment thereto of a suction hose or tube. The hose or tube in turn is connected to a suction source, such as those commonly found in operating rooms.

A collector **900** in accordance with a first such embodiment is shown in FIGS. 10a and 10b, wherein a perspective view of the collector **900** used to collect cut bone from a kerrison rongeur for harvesting is illustrated in a generally vertical orientation and includes a suction tube **950** attached to a suction port **952** located on the cap **905** of the collector **900.** FIG. 10b is a side plan view of the collector **900** that perhaps better shows in a close-up, cross-sectional view, the configuration of the intake port **913** of the collector **900** within which a distal tip of a kerrison rongeur is received. In accordance with this embodiment, the intake port includes one or more scrapers located within the interior thereof. Additionally, the intake port **913** itself forms a "hawk bill" scraping tip. The scrapers and scraping tip are used to dislodge and scrape bone from the kerrison rongeur, with the suction applied through the suction tube drawing into the collector the dislodged and scraped bone. The bone is caught and retained within an interior containment space of the container body **901** for later harvesting and use.

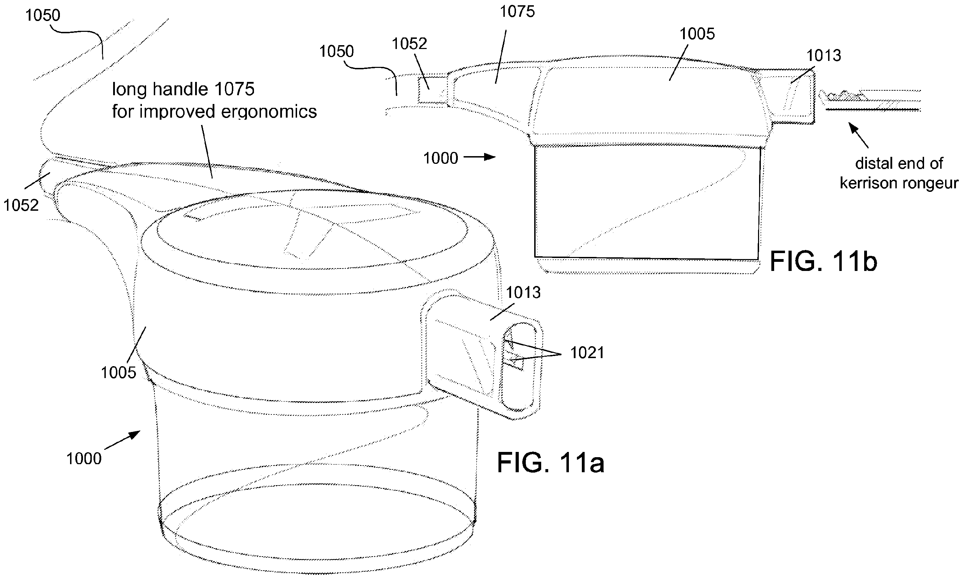

FIG. 11a is a perspective view of a collector **1000** used to collect cut bone from a kerrison rongeur for harvesting in accordance with yet another embodiment of the present invention, wherein the collector **1000** is illustrated in a generally vertical orientation and includes a suction tube **1050** attached to a suction port **1052** located on the cap **1005** of the collector **1000.** FIG. 11b is a side plan view of the collector **1000.**

Unlike collector **900,** the cap **1005** of collector **1000** forms a long, ergonomic handle **1075** for gripping where the suction port **1052** is located. Like collector **900,** the intake opening **1013** of collector **1000** is formed in the cap **1005** and includes scrapers **1021** located within the interior thereof, as seen in FIG. 11a.

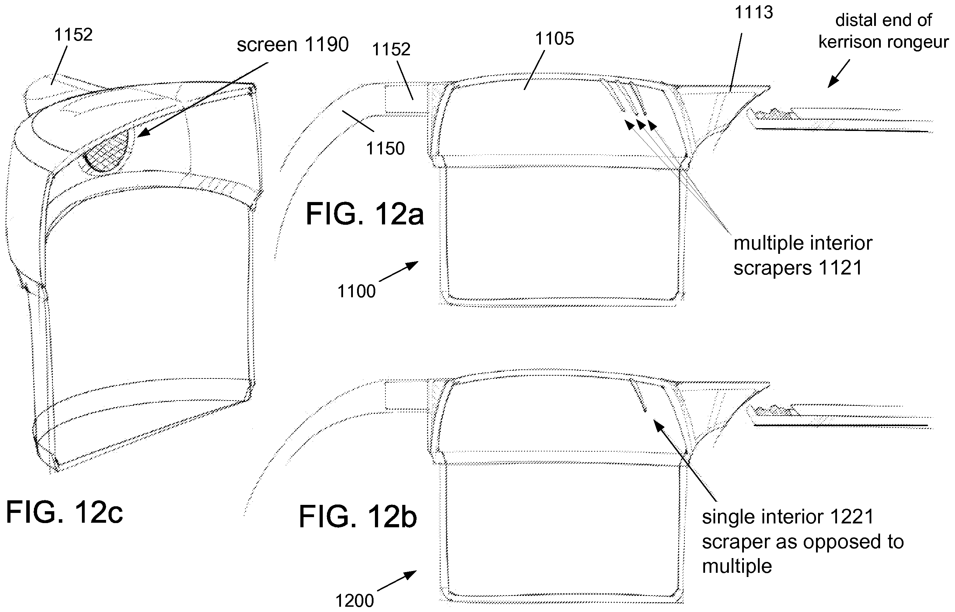

FIG. 12a is a side plan view, in cross-section, of a collector **1100** used to collect cut bone from a kerrison rongeur in accordance with yet another embodiment of the present invention, wherein the collector **1100** is illustrated in a generally vertical orientation and includes a suction tube **1150** attached to a suction port **1152** located on the cap **1105.** As shown in FIG. 12a, the collector **1100** includes multiple interior scrapers **1121** extending from the cap **1105** downwardly within the interior thereof for dislodging bone from the distal end of a kerrison rongeur.

FIG. 12b is a side plan view in cross-section of another collector **1200** similar to the collector **1100** of FIG. 12a, and differing only in that a single interior scraper **1221** is provided rather than a plurality of scrapers.

FIG. 12c is a perspective view in cross-section of a portion of the collector **1100** of FIG. 12a illustrating a porous screen mesh or screen **1190** for filtering of outflow from the collector **1100** through the suction tube **1150** so that precious bone is not inadvertently suctioned away from the collector **1100.** Indeed, a screen is preferred as the collector is most likely will be accidentally tipped over onto its side at some point during its use such that bone would be suctioned away if not for the screen.

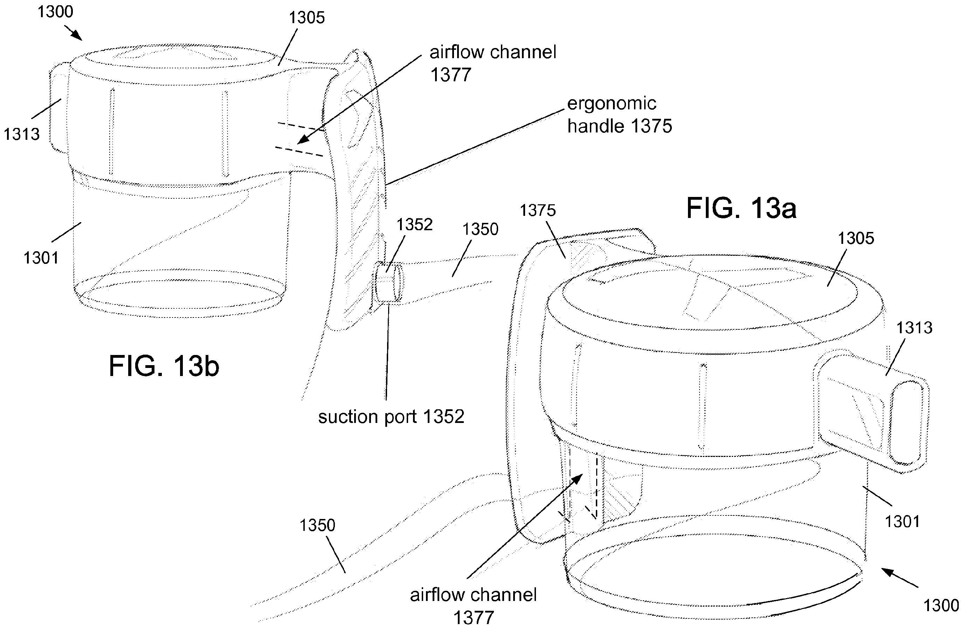

FIG. 13a is a perspective view of a collector **1300** used to collect cut bone from a kerrison rongeur for harvesting in accordance with yet another embodiment of the present invention, wherein the collector **1300** is illustrated in a generally vertical orientation. FIG. 13b is another perspective view of an opposite side of the collector **1300** as seen in FIG. 13a. The collector **1300** includes a suction tube **1350** attached to a suction port **1352** located at a distal end of a handle **1375** that, itself, is located proximate the bottom of the collector **1300.** An airflow channel **1377** is defined within and extends through the interior of the handle **1375** between the suction port **1313** and an interior containment space of the container body 1301 (which is represented in phantom in FIGS. 13a and 13b). As shown in FIG. 13a and 13b, the handle **1375** and suction port are part of the cap **1305,** which is removably attached to the container body **1301.** Intake port **1313** is also part of the cap **1305.**

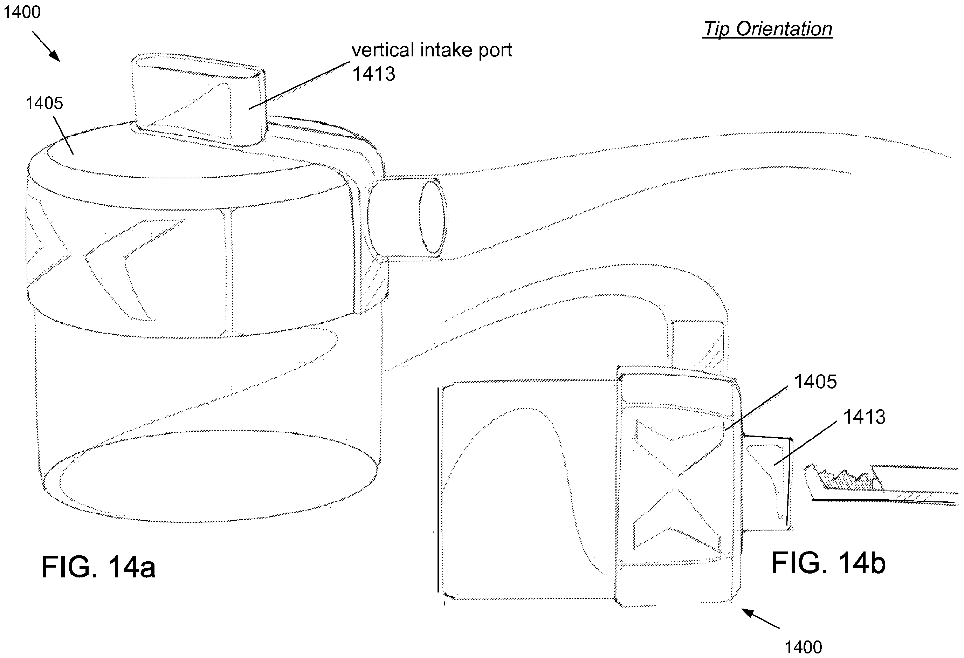

FIG. 14a is a perspective view of a collector **1400** used to collect cut bone from a kerrison rongeur in accordance with yet another embodiment of the present invention, wherein the collector **1400** is illustrated in a generally vertical orientation. FIG. 14b is a perspective view illustrating a use of the collector **1400** in collecting bone from a kerrison rongeur for harvesting, wherein the collector **1400** is illustrated in a generally horizontal orientation with the distal end of the kerrison rongeur about to be received through an intake port **1413** of the collector **1400** that is located on the top of the cap **1405** of the collector **1400.**

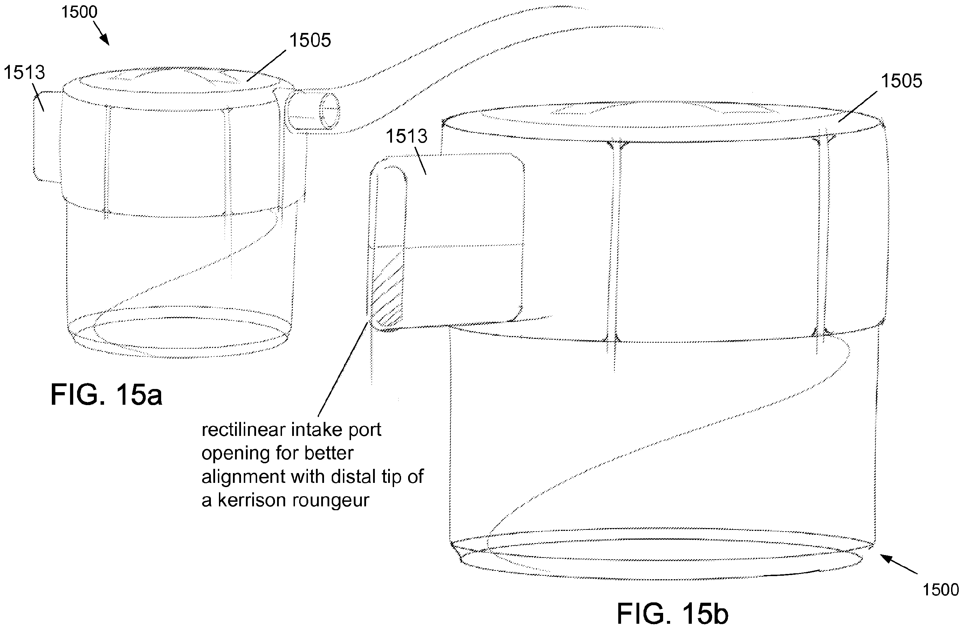

FIG. 15a is a perspective view of a collector **1500** used to collect cut bone from a kerrison rongeur in accordance with yet another embodiment of the present invention, wherein the collector **1500** is illustrated in a generally vertical orientation. FIG. 15b is another perspective view of an opposite side of the collector **1500** as seen in FIG. 15a, wherein an intake port **1513** of the collector **1500** is better seen. The intake port **1513** is located on a side of the cap **1505** of the collector **1500.**

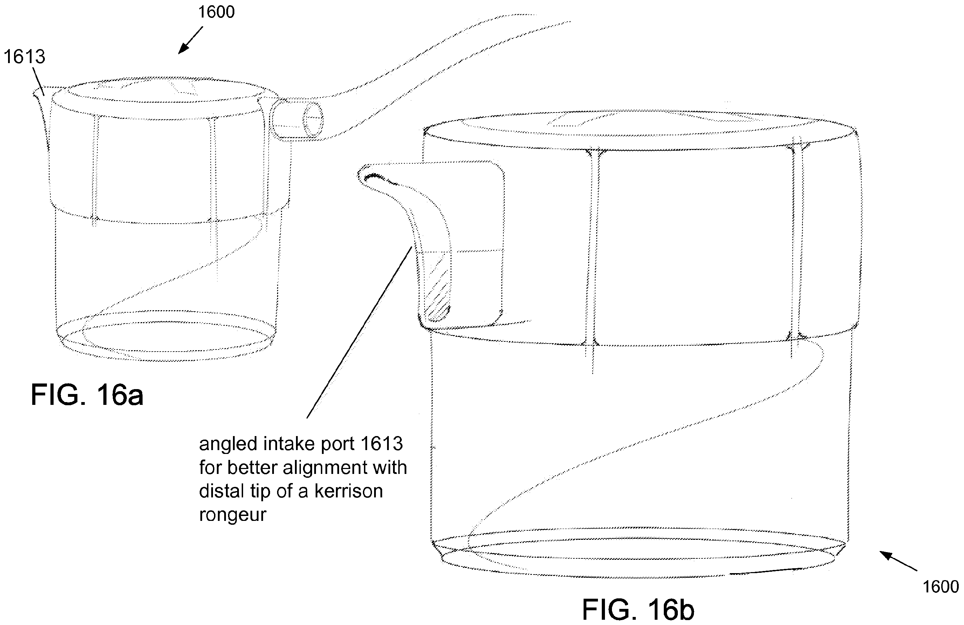

FIG. 16a is a perspective view of a collector **1600** used to collect cut bone from a kerrison rongeur in accordance with yet another embodiment of the present invention, wherein the collector **1600** is illustrated in a generally vertical orientation. FIG. 16b is another perspective view of an opposite side of the collector **1600** as seen in FIG. 16a, wherein an intake port **1613** of the collector **1600** is better seen.

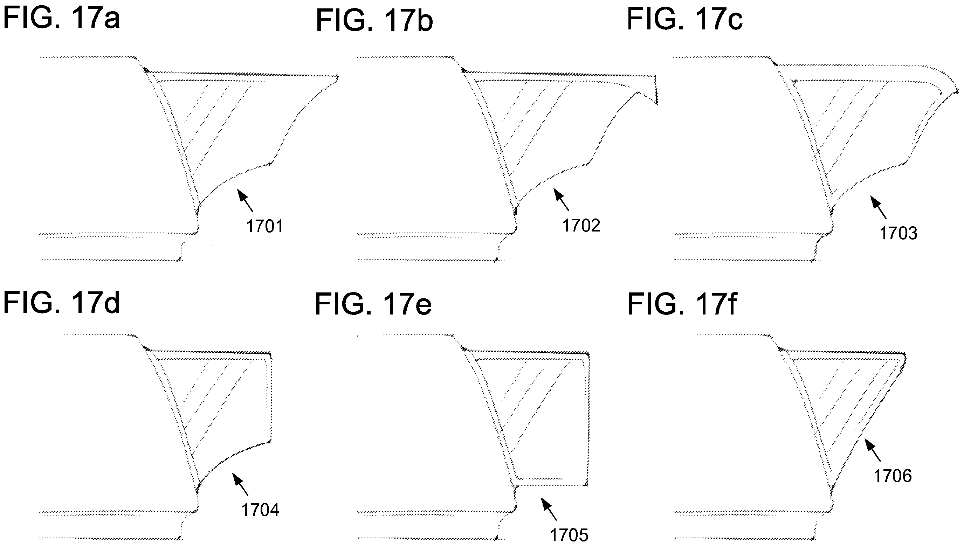

FIG. 17a illustrates, in a cross-sectional view, a profile of an intake port **1701** for a collector in accordance with various embodiments of the present invention, which intake port has a rounded, angled tip.

FIG. 17b illustrates, in a cross-sectional view, a profile of an intake port **1702** for a collector in accordance with various embodiments of the present invention, which intake port has a rounded, angled "hawk bill" scraping tip.

FIG. 17c illustrates, in a cross-sectional view, a profile of an intake port **1703** for a collector in accordance with various embodiments of the present invention, which intake port has a rounded, angled round hawk bill scraping tip.

FIG. 17d illustrates, in a cross-sectional view, a profile of an intake port **1704** for a collector in accordance with various embodiments of the present invention, which intake port has a rounded, rectilinear tip.

FIG. 17e illustrates, in a cross-sectional view, a profile of an intake port **1705** for a collector in accordance with various embodiments of the present invention, which intake port has a rectilinear tip.

FIG. 17f illustrates, in a cross-sectional view, a profile of an intake port **1706** for a collector in accordance with various embodiments of the present invention, which intake port has an angled tip.

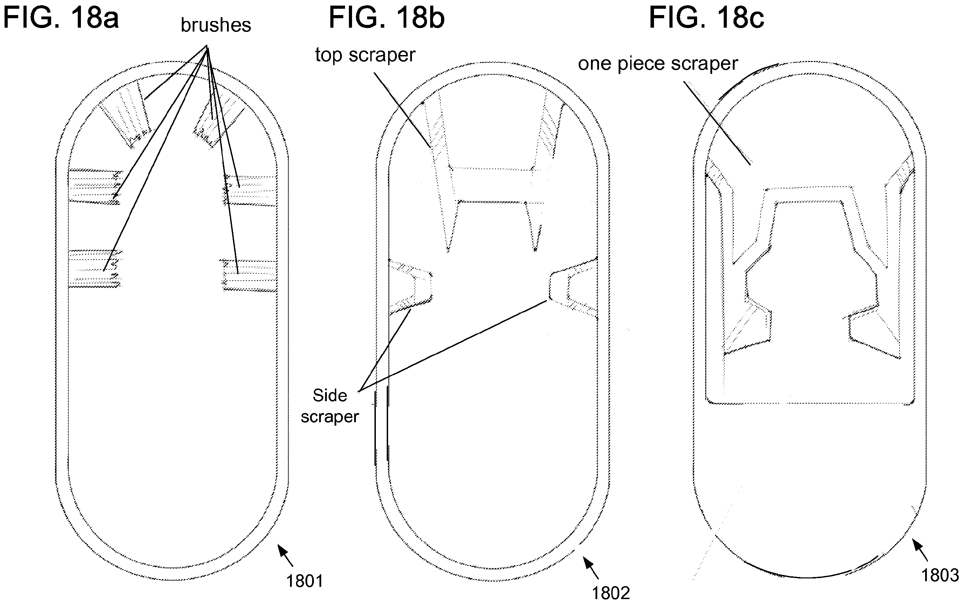

FIG. 18a illustrates in plan view an opening of an intake port **1801** for a collector in accordance with various embodiments of the present invention, which intake port includes multiple sets of bristle scrapers mounted on a top and sides of the port.

FIG. 18b illustrates in plan view an opening of an intake port **1802** for a collector in accordance with various embodiments of the present invention, which intake port includes side scrapers combined with one large top scraper.

FIG. 18c illustrates in plan view an opening of an intake port **1803** for a collector in accordance with various embodiments of the present invention, which intake port includes a single large top mounted scraper that scraps both sides and top simultaneously, and which port has a narrowed entrance to improve scraping efficiency upon insertion of a kerrison rongeur.

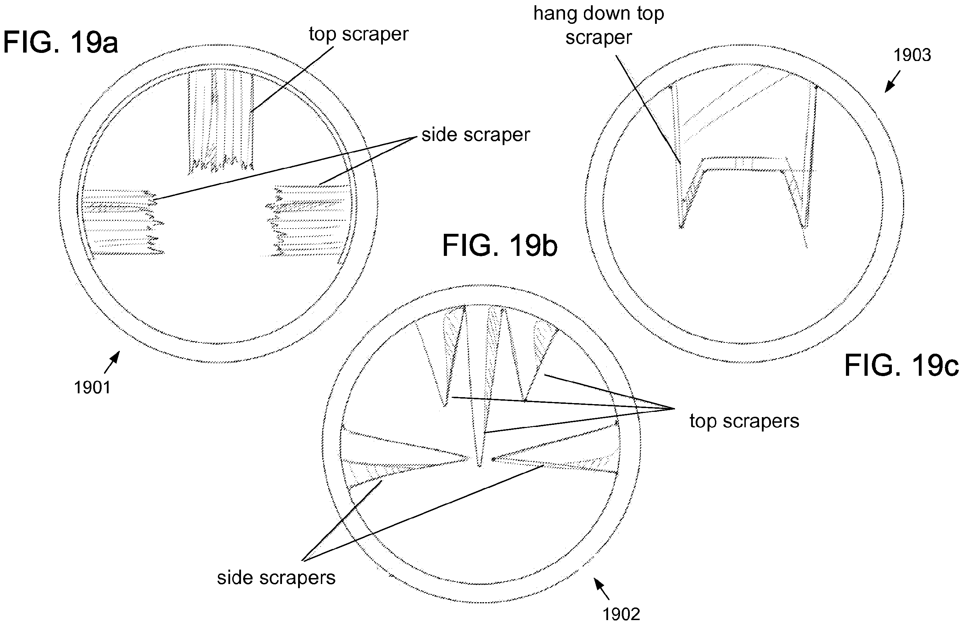

FIG. 19a illustrates in plan view a generally oval (preferably circular) intake port **1901** of for a collector in accordance with various embodiments of the present invention, which intake port includes top and side mounted scrapers.

FIG. 19b illustrates in plan view a generally oval (preferably circular) intake port **1902** for a collector in accordance various embodiments of the present invention, which intake port includes multiple top scrapers combined with side scrapers.

FIG. 19c illustrates in plan view a generally oval (preferably circular) intake port **1903** for a collector in accordance with various embodiments of the present invention, which intake port includes a single, top mounted scraper.

### More Embodiments and Prototypes

Still another collector in accordance with an embodiment of the present invention is shown in a perspective view in FIG. 20a. As shown therein, the intake port **2013** comprises a squeezable and springy material capable of being squeezed by hand. When the distal end of a kerrison rongeur is received within the intake port **2013,** the intake port is manually squeezed such that scrapers located within the intake port **2013** engage and dislodge any cut bone found within the cutting area of the kerrison rongeur. The locations, types, and configurations of the scrapers may be in accordance with the disclosures of scrapers found hereinabove.

FIG. 21a illustrates a table listing values of properties giving rise to some possible variations between some embodiments of the invention, and FIG. 21b illustrates a table listing some possible combinations of some of the elements of the table of FIG. 21a. In this respect, the first column of the table of FIG. 21a lists elements that maybe used in one or more embodiments. The listed elements include a brush; a soft wiper (i.e., soft scraper); a hard wiper (i.e., hard scraper); pressure (air); and suction. Each of these elements represents a way of removing or dislodging cut matter from a tip of a rongeur. The second column lists paths of movement of the tip of the rongeur relative to the element for the brush and different wipers. The third column lists possible orientation to the path. Exemplary combinations of some of these elements, including the brush and wipers, are set forth in FIG. 21b. FIGS. 22a through 22g are perspective views of tips and scrapers showing various paths and orientations.

A perspective view of a collector **2300** in accordance with yet another embodiment of the invention is illustrated in FIG. 23.

A perspective view of a collector **2400** in accordance with yet another embodiment of the invention is illustrated in FIGS. 24a-24f, wherein: FIG. 24a is a top view of the cap of the collector **2400;** FIG. 24b is a side elevational view of the collector **2400;** FIG. 24c is another side elevational view of the collector **2400;** FIG. 24d is a top perspective side view of the collector **2400;** FIG. 24e is a bottom perspective side view of the collector **2400;** and FIG. 24f is a perspective view of the scraper of the collector **2400** located under and attached to the cap, as seen through the opening in the cap in FIGS. 24c and 24e.

A perspective view of a collector **2500** in accordance with yet another embodiment of the invention is illustrated in FIGS. 25a-25f. A portion **2580** of the end of a kerrison rongeur including the tip is also illustrated relative to the container **2500** in FIGS. 25b-25f.

A perspective view of two collectors **2600,2650** each in accordance with another respective embodiment of the invention are illustrated in FIGS. 26a-24f. The two collectors **2600,2650** are structurally the same with the exception that brush **2610** of collector **2600** comprises bristles of the same length, whereas brush **2660** of collector **2650** has varying length bristles. In particular, FIG. 26a is a side-by-side elevational view of the tops of the collectors **2600,2650** looking through the ports (wide side openings) in the caps thereof at the brushes **2610,2660,** where the varying length bristles of brush **2660** can be seen. FIG. 26b is a perspective view of the caps of the collectors **2600,2652** after the caps have been unscrewed from the containers and placed upside down on a surface to expose views of the brushes **2610,2660.** As perhaps best shown in FIG. 26b, the brushes are secured to a generally semicircular area of the underside of the respective caps. Continuing with reference to collectors **2600,2650,** FIG. 26c is a side-by-side top perspective view of these collectors, and FIG. 26d is a side elevational view of collector **2650** being held with a left hand. The varying length of the bristles of brush **2660** also are seen in FIG. 26d as well.

FIGS. 27-28b demonstrate insertion of the tip of a kerrison rongeur through the port and into the interior of the cap of the collector **2600** in engagement with the brush **2610.** Specifically, FIG. 27 shows movement of the tip in the direction of arrow **A** so as to extend through the port; and FIGS. 28a and 28b each shows the tip being receive within the cap of the collector **2600** in engagement with the brush **2610.**

A perspective view of a collector **2900** in accordance with yet another embodiment of the invention is illustrated in FIG. 29 and comprises a container **2905** and cap **2910.** The cap includes a plunger that is spring-biased into an open position by spring **2942.** A scraper is located on the plunger inside the cap **2910** and, when the plunger is moved into the closed position by pressing on tab **2940** with a finger or thumb, the scraper (not shown) moves across the cutting area of a rongeur tip that has been inserted through port **2948** so as to dislodge matter carried on the rongeur in the cutting area.

FIGS. 30-34b show a prototype collector **3000** as represented by collector **2900** of FIG. 29. In particular, FIG. 30 shows the collector **3000** held in a person's left hand with thumb on the tab **3040** of the plunger. An alternative method of holding the collector is with the right hand, as demonstrated in FIGS. 31 and 32.

The plunger is biased by spring **3042** into an open position. The spring is attached and secured at one end by a pin to the plunger, and at the other end by a pin to the main body of the cap **3010.** In order to adjust the spring force, the pin can be placed in one of a plurality of holes formed in the cap **3010** as perhaps best seen in FIG. 30 and illustrated in representative collector **2900** of FIG. 29. The cap **3010** includes an port **3048** physically configured to receive the tip of a kerrison rongeur there through, as seen in FIGS. 33a and 33b.

When inserted through the port **3048,** the distal end **3045** of the tip of the rongeur contacts a stop **3030,** which is best seen in FIG. 34b. Indeed, in FIG. 34b the tip is shown having been inserted through port **3048** of cap **3010** and into abutment with stop **3030,** whereat movement of the plunger by pressing on tab **3040** will force the scraper (in the form of a brush **3025**) directly through the cutting area of the tip, thereby dislodging matter in the cutting area.

In accordance with preferred embodiments of the present invention, a patient advantageously is afforded his or her own bone for the fusion when a collector is used to harvest bone cut from the patient using a kerrison rongeur. For example, Lamina chips are clinically proven to have both osteoinductive and osteoconductive properties conducive for bone fusion. By using a patient's own bone, there is less chance of rejection, infection, and significant cost by not having to rely on bone substitute.

Based on the foregoing description, it will be readily understood by those persons skilled in the art that the present invention is susceptible of broad utility and application. Many embodiments and adaptations of the present invention other than those specifically described herein, as well as many variations, modifications, and equivalent arrangements, will be apparent from or reasonably suggested by the present invention and the foregoing descriptions thereof, without departing from the scope of the present invention. Accordingly, while the present invention has been described herein in detail in relation to one or more preferred embodiments, it is to be understood that this disclosure is only illustrative and exemplary of the present invention and is made merely for the purpose of providing a full and enabling disclosure of the invention. The foregoing disclosure is not intended to be construed to limit the present invention or otherwise exclude any such other embodiments, adaptations, variations, modifications or equivalent arrangements, the present invention being limited only by the claims appended hereto.

## Claims

1. A collector (200) comprising a container (201) and a removable cap (205) attached to the container (201), the cap (205) defining an opening (213) dimensioned for receiving the tip of a Kerrison rongeur therethrough, and further comprising a scraper (211) attached to an underside of the cap (205) such that extension of the rongeur through the opening (213) dislodges material carried in a cutting area of the rongeur, which dislodged material is received within an interior space of the container (201).

2. The collector (200) of claim 1, wherein the scraper (211) extends toward the interior containment space.

3. The collector (200) of claim 1, wherein the opening (213) is located in a sidewall of the cap.

4. The collector (200) of claim 1, wherein the scraper (211) is the only scraper in the collector.

5. The collector (200) of claim 1, wherein the opening (213) defined by the cap (205) is the only opening for receiving the tip of the rongeur therethrough for dislodging, by the scraper (211), material carried in the cutting area of the rongeur.

6. The collector (200) of claim 1, wherein the container (201) comprises an opaque wall portion (103) and a transparent wall portion (105).

7. The collector (200) of claim 1, further comprising one or more scrapers (211) for dislodging material carried in the cutting area of the rongeur.

8. The collector (200) of claim 7, wherein a said scraper (211) comprises bristles.

9. The collector (200) of claim 7, wherein a said scraper (211) comprises a brush.

10. The collector (200) of claim 7, wherein a said scraper (211) comprises a protuberance.

11. The collector (200) of claim 7, wherein a said scraper (211) comprises a barb.

12. The collector (200) of claim 7, wherein a said scraper (211) comprises a finger.

13. The collector (200) of claim 7, wherein a said scraper(211) is flexible and resilient, and is sufficiently rigid so as to hold form when not engaged by the distal end of the rongeur while deflecting and bending upon abutment by and engagement with the distal end of the rongeur.

14. The collector (200) of claim 7, wherein a said scraper (211) is made from polypropylene.

15. A method of using a collector (200) of any of the foregoing claims to collect cut bone from a Kerrison rongeur.

## Patentansprüche

1. Sammler (200), der einen Behälter (201) und einen an dem Behälter (201) angeordneten, abnehmbaren Deckel (205) aufweist, wobei der Deckel (205) eine Öffnung (213) zur Aufnahme der Spitze eines Kerrison Rongeurs durch diese definiert, und ferner einen Abstreifer (211) umfasst, der an einer Unterseite des Deckels (205) angeordnet ist, sodass eine Verlängerung des Rongeurs durch die Öffnung (213) das in einem Schneidbereich des Rongeurs getragene Material ablöst, wobei das abgelöste Material in einem Innenraum des Behälters (201) aufgenommen wird.

2. Sammler (200) nach Anspruch 1, **dadurch gekennzeichnet, dass** der Abstreifer (211) sich in Richtung eines inneren Sicherheitsraums erstreckt.

3. Sammler (200) nach Anspruch 1, **dadurch gekennzeichnet, dass** die Öffnung (213) in einer Seitenwand des Deckels angeordnet ist.

4. Sammler (200) nach Anspruch 1, **dadurch gekennzeichnet, dass** der Abstreifer (211) der einzige Abstreifer im Sammler ist.

5. Sammler (200 nach Anspruch 1, **dadurch gekennzeichnet, dass** die durch den Deckel (205) definierte Öffnung (213) die einzige Öffnung zur Aufnahme der Spitze des Kerrison Rongeurs durch diese zum Ablösen, durch den Abstreifer (211), von im Schneidbereich des Rongeurs getragenem Material ist.

6. Sammler (200) nach Anspruch 1, **dadurch gekennzeichnet, dass** der Behälter (201) einen opaken Wandabschnitt (103) und einen transparenten Wandabschnitt (105) aufweist.

7. Sammler (200) nach Anspruch 1, **dadurch gekennzeichnet, dass** der Sammler (200) ferner einen oder mehrere Abstreifer (211) zum Ablösen von im Schneidbereich des Rongeurs getragenem Material ist.

8. Sammler (200) nach Anspruch 7, **dadurch gekennzeichnet, dass** ein genannter Abstreifer (211) Borsten aufweist.

9. Sammler (200) nach Anspruch 7, **dadurch gekennzeichnet, dass** ein genannter Abstreifer (211) eine Bürste umfasst.

10. Sammler (200) nach Anspruch 7, **dadurch gekennzeichnet, dass** ein genannter Abstreifer (211) Vorsprünge aufweist.

11. Sammler (200) nach Anspruch 7, **dadurch gekennzeichnet, dass** ein genannter Abstreifer (211) einen Widerhaken umfasst.

12. Sammler (200) nach Anspruch 7, **dadurch gekennzeichnet, dass** ein genannter Abstreifer (211) einen Finger umfasst.

13. Sammler (200) nach Anspruch 7, **dadurch gekennzeichnet, dass** ein genannter Abstreifer flexibel und elastisch und ausreichend beigesteif ist, um die Form zu halten, wenn er nicht am distalen Ende des Rongeurs in Eingriff steht während er sich am Widerlager durch einen Eingriff mit dem distalen Ende des Rongeurs auslenkt und biegt.

14. Sammler (200) nach Anspruch 7, **dadurch gekennzeichnet** das ein genannter Abstreifer (211 aus Polypropylen gefertigt ist.

15. Verwendung des Sammlers (200) nach einem der vorhergehenden Ansprüche zum Sammeln von geschnittenen Knochen von einem Kerrison Rongeur.

## Revendications

1. Collecteur (200) comprenant un récipient (201) et un capuchon amovible (205) attaché au récipient (201), le capuchon (205) définissant une ouverture (213) dimensionnée pour recevoir la pointe d'un rongeur de Kerrison à travers elle, et comprenant en outre un racleur (211) fixé à une face inférieure du capuchon (205) de telle sorte que l'extension du rongeur à travers l'ouverture (213) déloge le matériau porté dans une zone de coupe du rongeur, lequel matériau délogé est reçu dans un espace intérieur du récipient (201).

2. Collecteur (200) selon la revendication 1, dans lequel le racleur (211) s'étend vers l'espace de confinement intérieur.

3. Collecteur (200) selon la revendication 1, dans lequel l'ouverture (213) est située dans une paroi latérale du capuchon.

4. Collecteur (200) selon la revendication 1, dans lequel le racleur (211) est le seul racleur dans le collecteur.

5. Collecteur (200) selon la revendication 1, dans lequel l'ouverture (213) définie par le capuchon (205) est la seule ouverture pour recevoir la pointe du rongeur à travers elle pour déloger, au moyen du racleur (211), le matériau transporté dans la zone de coupe du rongeur.

6. Collecteur (200) selon la revendication 1, dans lequel le récipient (201) comprend une partie de paroi opaque (103) et une partie de paroi transparente (105).

7. Collecteur (200) selon la revendication 1, comprenant en outre un ou plusieurs racleurs (211) pour déloger le matériau transporté dans la zone de coupe du rongeur.

8. Collecteur (200) selon la revendication 7, dans lequel ledit racleur (211) comprend des poils.

9. Collecteur (200) selon la revendication 7, dans lequel ledit racleur (211) comprend une brosse.

10. Collecteur (200) selon la revendication 7, dans lequel ledit racleur (211) comprend une protubérance.

11. Collecteur (200) selon la revendication 7, dans lequel ledit racleur (211) comprend un ardillon.

12. Collecteur (200) selon la revendication 7, dans lequel ledit racleur (211) comprend un doigt.

13. Collecteur (200) selon la revendication 7, dans lequel ledit racleur (211) est flexible et élastique, et est suffisamment rigide pour maintenir sa forme lorsqu'il n'est pas engagé par l'extrémité distale de la goupille tout en déviant et se pliant lors de la mise en butée par et l'engagement avec l'extrémité distale du rongeur.

14. Collecteur (200) selon la revendication 7, dans lequel ledit racleur (211) est en polypropylène.

15. Procédé d'utilisation d'un collecteur (200) selon l'une quelconque des revendications précédentes pour collecter de l'os coupé à partir d'un rongeur de Kerrison.
